# EUROPEAN PATENT SPECIFICATION

(11) **EP 4 633 582 B1**
(45) Date of publication and mention of the grant of the patent: **02.12.2026**
(21) Application number: 23837443.3
(22) Date of filing: 08.12.2023
(51) Int. Cl.: A61K 8/06, A61K 8/25, A61K 8/49, A61K 8/58, A61K 8/73, A61K 8/893, A61Q 1/00, A61Q 1/02, A61K 8/34

(54) **COSMETIC EMULSION COMPOSITION COMPRISING SPHERICAL HYDROPHOBIC SILICA AEROGEL FOR WATERY FRESH SENSATION**
KOSMETISCHE EMULSIONSZUSAMMENSETZUNG MIT SPHÄRISCHEM HYDROPHOBEM SILICIUMDIOXIDAEROGEL FÜR WASSERHALTIGES ERFRISCHUNGSEMPFINDEN
COMPOSITION D'ÉMULSION COSMÉTIQUE COMPRENANT UN AÉROGEL DE SILICE HYDROPHOBE SPHÉRIQUE POUR SENSATION D'EAU FRAÎCHE

(30) Priority: 15.12.2022 JP 2022200279; 27.01.2023 FR 2300776
(43) Date of publication of application: 22.10.2025
(73) Proprietor: L'OREAL, 75008 Paris (FR); Suda, Mari, Kawasaki-shi, Kanagawa 2130012 (JP); Ogami, Kazunori, Kawasaki-shi, Kanagawa 2130012 (JP); Dumrongkulchart, Napatsorn, Kawasaki-shi, Kanagawa 213-0012 (JP)
(72) Inventor: SUDA, Mari, Kawasaki-shi, Kanagawa 2130012 (JP); OGAMI, Kazunori, Kawasaki-shi, Kanagawa 2130012 (JP); DUMRONGKULCHART, Napatsorn, Kawasaki-shi, Kanagawa 2130012 (JP)
(74) Representative: Casalonga
(86) International application number: PCT/JP2023/044953
(87) International publication number: WO 2024/128300

(56) References cited:
- WO-A1-2022/199974
- DATABASE GNPD [online] MINTEL; 25 January 2023 (2023-01-25), ANONYMOUS: "Nude Hyaluronic Tinted Serum", XP093071786, retrieved from https://www.gnpd.com/sinatra/recordpage/10529992/ Database accession no. 10529992

## Description

### TECHNICAL FIELD

The present invention relates to a cosmetic composition in the form of an emulsion comprising at least one spherical hydrophobic silica aerogel for a watery fresh sensation.

### BACKGROUND ART

Imparting a watery and fresh texture to keratinous substances, such as skin, is one of the key features of cosmetic products, in particular skin cosmetic products. Emulsions are commonly employed as the form of liquid foundation products, since they are pleasant to use due to the feeling of freshness and the moisture that the aqueous phase can provide.

Spherical silica silylate aerogel particles have recently been developed, and it has been reported that these particles provide emulsion compositions with a long-lasting stability.

For example, JP-A-2021-102558 discloses a cosmetic composition in the form of a W/O emulsion, comprising at least one spherical hydrophobic silica aerogel and at least one ester oil selected from liquid esters of saturated or unsaturated, linear or branched C₁-C₂₆ aliphatic monoacid or polyacids and of saturated or unsaturated, linear or branched C₁-C₂₆ aliphatic monoalcohol or polyalcohol.

Also, JP-A-2021-102559 discloses a cosmetic composition comprising (i) at least one spherical hydrophobic silica aerogel and (ii) at least one composite silica particle and/or at least one hollow silica particle.

WO2022199974 A1 discloses a cosmetic water-in-oil emulsion composition comprising at least one aqueous phase and at least one oily phase, comprising a spherical hydrophobic silica aerogel (silica silylate), a thickening auxiliary (PEG-10 dimethicone), a film-forming polymer (trimethoxysiloxysilicate) and polyols (glycerol, propylene glycol).

However, there is still a demand of for cosmetic compositions which can provide keratinous substances, such as skin, with a moist, watery, and fresh sensation.

### DISCLOSURE OF INVENTION

An objective of the present invention is to provide a cosmetic composition which can provide a watery and fresh sensation with long-lasting moisture which has a good stability.

The above objective can be achieved by an emulsion composition having at least one aqueous phase and at least one oily phase, comprising:
(a) at least one spherical hydrophobic silica aerogel;
(b) at least one thickening aid selected from polyoxyalkylenated silicone and alkyl or alkylene carbonate;
(c) at least one film-forming polymer;
(d) at least one polyol in an amount of 6% by weight or more relative to the total weight of the composition; and
(e) at least one anionic polymer,
wherein the aqueous phase is present in an amount of 40% by weight or more relative to the total weight of the composition.

The spherical hydrophobic silica aerogel may be a spherical hydrophobic aerogel of silica silylate.

The spherical hydrophobic silica aerogel may have an average circularity determined by an image analysis method of 0.8 or more, and preferably 0.82 or more, and of less than 1, preferably 0.99 or less, more preferably 0.98 or less, even more preferably 0.97 or less, still even more preferably 0.96 or less, and most preferably 0.95 or less.

The spherical hydrophobic silica aerogel may have an oil-absorbing capacity, measured at the wet point, of 2 ml/g or more, preferably 3 ml/g or more, more preferably 4 ml/g or more, and most preferably from 5 ml/g or more, and of 12 ml/g or less, preferably 11 ml/g or less, more preferably 10 ml/g or less, and most preferably 8 ml/g or less.

The film-forming polymer may be selected from silicone resin.

The film-forming polymer is selected from silicone resin of MQ type.

The polyol may comprise at least one diol and at least one polyol having three or more -OH functions, in particular triol, in combination.

The polyol may comprise at least 3% by weight of at least one diol and at least 3% by weight of at least one polyol having three or more -OH functions, in particular triol, in combination, relative to the total weight of the composition.

A number average molecular weight of the anionic polymer may range from 1,000 to 1,000,000, preferably from 2,000 to 500,000, and more preferably from 3,000 50,000.

The anionic polymer may be selected from the group consisting of polysaccharides such as alginic acid, hyaluronic acid and derivatives thereof, and cellulose polymers (e.g., carboxymethylcellulose), anionic (co)polyaminoacids such as (co)polyglutamic acids, (co)poly(meth)acrylic acids, (co)polyamic acids, (co)polystyrene sulfonate, (co)poly(vinyl sulfate), dextran sulfate, chondroitin sulfate, (co)polymaleic acids, (co)polyfumaric acids, maleic acid (co)polymers.

The anionic polymer may be selected from hyaluronic acid and derivatives thereof, and a salt thereof; and preferably hyaluronic acid and acetylated hyaluronic acid, and a salt thereof.

The thickening aid may be selected from dimethicone copolyols comprising polyethyleneoxy moiety.

The thickening aid may be selected from alkyl or alkylene carbonate wherein the alkylene chain(s) of the alkylene carbonate(s) and/or the alkyl radical(s) of the alkyl carbonate(s) comprise(s) from 1 to 6 carbon atoms.

The aqueous phase may comprise at least one monoalcohol.

The cosmetic composition according to the present invention may be a skin makeup or skin care composition, and preferably a skin makeup composition, in particular a foundation.

The present invention also relates to a cosmetic process for a keratin substance such as skin, comprising applying the cosmetic composition according to the present invention to the keratin substance.

### BEST MODE FOR CARRYING OUT THE INVENTION

After diligent research, the inventors have discovered that a composition in the form of an emulsion comprising the components (a) to (e) according to the present invention can provide a watery and fresh sensation with long-lasting moisture which has a good stability, and thus completed the invention.

Thus, the composition according to the present invention is an emulsion composition having at least one aqueous phase and at least one oily phase, comprising:
(a) at least one spherical hydrophobic silica aerogel;
(b) at least one thickening aid selected from polyoxyalkylenated silicone and alkyl or alkylene carbonate;
(c) at least one film-forming polymer;
(d) at least one polyol in an amount of 6% by weight or more relative to the total weight of the composition; and
(e) at least one anionic polymer,
wherein the aqueous phase is present in an amount of 40% by weight or more relative to the total weight of the composition.

Hereinafter, the composition according to the present invention will be explained in a more detailed manner.

### [Composition]

The composition of the present invention is in the form of as emulsion comprising an aqueous phase and an oily phase. The emulsion may be O/W or W/O form. In one preferred embodiment of the present invention, the composition is in the form of W/O emulsion.

The term "W/O emulsion" or "water-in-oil emulsion" means any macroscopically homogeneous composition comprising a continuous fatty or oily phase and aqueous or water phases in the form of droplets dispersed in the said fatty or oily phase. The term "O/W emulsion" or "oil-in-water emulsion" means any macroscopically homogeneous composition comprising a continuous aqueous or water phase and fatty or oily phases in the form of droplets dispersed in the aqueous or water phase.

The composition is a cosmetic composition, preferably a cosmetic composition for a keratin substance, preferably a skin makeup or a skin care composition, and more preferably a skin makeup composition. The keratinous substance here means a material containing keratin as a main constituent element, and examples thereof include the skin, scalp, lips, and the like. Preferably, the composition of the present invention is used for skin, and more preferably facial skin.

In one preferred embodiment, the composition according to the present invention can be used as a liquid foundation, a make-up base, and a skin makeup cream composition, and in particular a liquid foundation. The composition can be in the form of a lotion, a milky lotion, a cream, a liquid gel, a paste, or a serum.

The composition according to the present invention can provide keratinous substances, such as skin, with moist, watery, and fresh sensation. In particular, the composition according to the present invention can provide keratinous substances with "water-splashing" sensation. The expression of "water-splashing" sensation here means a feel like a splash or a disperse of water on the keratinous substances during an application. Such a feel can give consumers a fresh sensation.

The composition according to the present invention comprises (a) at least one spherical hydrophobic silica aerogel; (b) at least one thickening aid selected from polyoxyalkylenated silicone and alkyl or alkylene carbonate; (c) at least one film-forming polymer; (d) at least one polyol; and (e) at least one anionic polymer. The ingredients in the composition will be described in a detailed manner below.

### (Spherical Hydrophobic Silica Aerogel)

The composition according to the present invention comprises at least one spherical hydrophobic silica aerogel. Two or more types of spherical hydrophobic silica aerogels may be used in combination. Thus, a single type of the spherical hydrophobic silica aerogel or a combination of different types of the spherical hydrophobic silica aerogels may be used.

Aerogels are materials with high porosity. Herein, silica aerogels refer to a solid silica with a porous structure generally obtained by replacing medium included in wet silica aerogels with air by drying them while a solid network structure of the silica is maintained. The porosity represents the amount of air contained in an apparent volume of a material by a volume percentage. The spherical hydrophobic silica aerogel of the present invention may have a porosity of 60% or more, preferably 70% or more, and more preferably 80% or more.

The hydrophobic silica aerogel of the present invention is characterized in that the shape of each of the particles is spherical. Due to this spherical shape, the hydrophobic silica aerogel can provide cosmetic compositions with good smoothness. The spherical degree of the hydrophobic silica aerogel may be determined by an average circularity.

The spherical hydrophobic silica aerogel of the present invention may have an average circularity of 0.8 or more, and preferably 0.82 or more. The spherical hydrophobic silica aerogel may have an average circularity of less than 1, preferably 0.99 or less, more preferably 0.98 or less, even more preferably 0.97 or less, still even more preferably 0.96 or less, and most preferably 0.95 or less.

The "average circularity" may be determined by an image analysis method. In particular, the "average circularity" may be an arithmetic mean of circularity obtained by image analysis of a scanning electron microscope (SEM) image of no less than 2000 aerogel particles observed at a magnification of 1000 by secondary electron detection using a scanning electron microscope (SEM).

The "circularity" of each aerogel particle is a value determined by the following formula: $C = 4 πS / {L}^{2}$ wherein C represents a circularity, S represents an area (projected area) of the aerogel particle in the image, and L represents a length of a periphery (perimeter) of the aerogel particle in the image. When the average circularity approaches 1, the shape of each of the particles becomes more spherical.

In the spherical hydrophobic silica aerogel of the present invention, the term "hydrophobicity" means that it is difficult for silica aerogel particles to disperse in water. More specifically, this term means that an aerogel phase and an aqueous phase are completely separated after 1 g of the silica aerogel particles and 100 g of ion-exchange water are added to a bottle, the bottle is agitated or stirred for ten or more seconds, and the bottle is left to stand. Therefore, in one particular embodiment of the present invention, the spherical hydrophobic silica aerogel does not exhibit a water absorption property.

The spherical hydrophobic silica aerogel that may be used according to the present invention is preferably of silylated silica type (INCI name: silica silylate). Most preferably, the spherical hydrophobic silica aerogel may be those described in JP-A-2014-088307, JP-A-2014-218433, or JP-A-2018-177620.

The hydrophobicity may be obtained by reacting a hydrophobizing agent with a silanol group represented by the following formula existing on the surface of silica:

≡Si-OH
wherein the symbol "=" represents the remaining three valences of the Si atom,
thereby converting the silanol group into a group represented by the following formula:

   (≡Si-O-)₍₄₋ₙ₎SiRₙ
wherein n is an integer of 1 to 3; each R is independently a hydrocarbyl group; and two or more R may be the same or different from each other, where n is 2 or more.

The hydrophobizing agent may be a silylating agent. Therefore, according to one preferred embodiment, in the spherical hydrophobic silica aerogel, the silica particles may be modified at the surface by silylation. As examples of the silylating agents, mention may be made of a treating agent having one of the following formulae (1) to (3).

Formula (1): RₙSiX₍₄₋ₙ₎

wherein n represents an integer of 1 to 3; R represents a hydrocarbyl group; X represents a group (i.e. a leaving group) which can leave a molecule by cleavage of bond with the Si atom in a reaction with a compound having a hydroxyl group; each R may be different where n is 2 or more; and each X may be different where n is 2 or less. 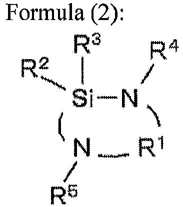 wherein R¹ represents an alkylene group; R² and R³ independently represent a hydrocarbyl group; and R⁴ and R⁵ independently represent a hydrogen atom or a hydrocarbyl group. 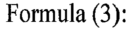 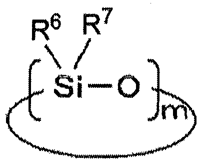 wherein R⁶ and R⁷ independently represent a hydrocarbyl group; m represents an integer of 3 to 6; each R⁶ may be different when there are two or more R⁶; and each R⁷ may be different when there are two or more R⁷.

In the above formula (1), R is a hydrocarbyl group, preferably a hydrocarbyl group having a carbon number of 1 to 10, more preferably a hydrocarbyl group having a carbon number of 1 to 4, and especially preferably a methyl group.

As examples of the leaving group represented by X, mention may be made of halogen atoms such as chlorine and bromine; alkoxy groups such as methoxy group and ethoxy group; groups represented by -NH-SiR₃ (wherein the definition of R is the same as that of R in the formula (1)).

Specific examples of the hydrophobizing agent represented by the above formula (1) include: chlorotrimethylsilane, dichlorodimethylsilane, trichloromethylsilane, monomethyltrimethoxysilane, monomethyltriethoxysilane, and hexamethyldisilazane.

Most preferably, chlorotrimethylsilane, dichlorodimethylsilane, trichloromethylsilane, and/or hexamethyldisilazane may be used from the viewpoint of favorable reactivity.

The number of bonds of the Si atom with the silanol group on the silica framework varies depending on the number (4-n) of the leaving group X. For example, if n is 2, the following bonding will occur:

(≡Si-O-)₂SiR₂.

If n is 3, the following bonding will occur:

≡Si-O-SiR₃.

In this manner, the silanol groups may be silylated, and thereby hydrophobization may be carried out.

In the above formula (2), R¹ may be an alkylene group, preferably an alkylene group having a carbon number of 2 to 8, and especially preferably an alkylene group having a carbon number of 2 to 3.

In the above formula (2), R² and R³ are independently a hydrocarbyl group, and the same preferable groups as those of R in the formula (1) can be raised. R⁴ represents a hydrogen atom or a hydrocarbyl group, and when it is a hydrocarbyl group, the same preferable groups as those of R in the formula (1) can be raised. When a gel of silica is treated with the compound (cyclic silazane) represented by the formula (2), cleavage of Si-N bonds will occur by the reaction with silanol groups, and therefore the following bonding will occur on the surface of the silica framework in the gel:

(≡Si-O-)₂SiR²R³.

**In** this way, the silanol group may be silylated by the cyclic silazanes of the above formula (2) as well, and thereby hydrophobization may be carried out.

Specific examples of the cyclic silazanes represented by the above formula (3) include hexamethylcyclotrisilazane, and octamethylcyclotetrasilazane.

In the above formula (3), R⁶ and R⁷ are independently a hydrocarbyl group, and the same preferable groups as those of R in the formula (2) can be raised. m represents an integer of 3 to 6. When a gel of silica is treated with the compound (cyclic siloxane) represented by the formula (3), the following bonding will occur on the surface of the silica framework in the gel:

(=Si-O-)₂SiR⁶R⁷.

In this way, silanol groups may be silylated by the cyclic siloxanes of the above formula (3) as well, and thereby hydrophobization may be carried out.

Specific examples of the cyclic siloxanes represented by the above formula (3) include hexamethylcyclotrisiloxane, octamethylcyclotetrasiloxane, and decamethylcyclopentasiloxane.

The spherical hydrophobic silica aerogel may be prepared by producing a sol of silica, turning the sol into a gel, aging the gel, washing the aged gel, replacing water in the washed gel with a solvent, treating the gel with a hydrophobizing agent, and dying the hydrophobized silica.

The spherical hydrophobic silica aerogel may have a specific surface area determined by BET method of 200 m²/g or more, preferably 400 m²/g or more, and more preferably 500 m²/g or more, and may have a specific surface area determined by BET method of 1,200 m²/g or less, preferably 1,000 m²/g or less, and more preferably 800 m²/g or less.

In the present invention, the "specific surface area determined by BET method" means a value determined by: drying a sample for measurement at 200°C for no less than three hours under a reduced pressure of no more than 1 kPa; thereafter measuring an adsorption isotherm of only a nitrogen adsorption side at liquid nitrogen temperature; and analyzing the adsorption isotherm by the BET method. The pressure range used for the analysis is a relative pressure of 0.1 to 0.25.

The spherical hydrophobic silica aerogel may have a pore volume determined by BJH method of 1 ml/g or more, preferably 2 ml/g or more, and more preferably 3 ml/g or more, and may have a pore volume determined by BJH method of 10 ml/g or less, preferably 8 ml/g or less, and more preferably 7 ml/g or less. The spherical hydrophobic silica aerogel may have a peak pore radius determined by BJH method of 5 nm or more, preferably 10 nm or more, and more preferably 12 nm or more, and may have a peak pore radius determined by BJH method of 50 nm or less, preferably 40 nm or less, and more preferably 30 nm or less.

The "pore volume determined by BJH method" refers to a pore volume which is derived from a pore having a pore radius of 1 nm to 100 nm obtained by analyzing, by the BJH method (Barrett, E. P.; Joyner, L. G.; Halenda, P. P., J. Am. Chem. Soc. 73, 373 (1951)), the adsorption isotherm of the nitrogen adsorption side obtained in the same manner as explained in the above "specific surface area determined by BET method". The "peak pore radius determined by BJT method" refers to a value of a pore radius which gives a peak in a pore distribution curve (volume distribution curve) which is plotted taking on the vertical axis differentiation of the cumulative pore volume by the logarithm of the pore radius obtained by analyzing, by the BJH method, the adsorption isotherm of the nitrogen adsorption side obtained in the same manner as above, and taking the pore radius on the horizontal axis.

The spherical hydrophobic silica aerogel may have an average particle size of 0.5 µm or more, preferably 1 µm or more, and more preferably 2 µm or more, and may have an average particle size by image analysis method of 30 µm or less, preferably 20 µm or less, and more preferably 15 µm or less.

The "average particle size" here can be measured by an image analysis method. Specifically, the value of "average particle size" is an arithmetic mean of equivalent circle diameters which can be obtained by image analysis of a scanning electron microscope (SEM) image of, for example, no less than 2000 aerogel particles observed at a magnification of 1000 by secondary electron detection using a scanning electron microscope (SEM). The "equivalent circle diameter" of each aerogel particle is a diameter of a circle having an area equal to the area (projected area) of the aerogel particle in the image.

Preferably, the spherical hydrophobic silica aerogel may have an oil-absorbing capacity, which can be measured at the wet point, of 2 ml/g or more, preferably 3 ml/g or more, more preferably 4 ml/g or more, and most preferably from 5 ml/g or more, and may have an oil-absorbing capacity, measured at the wet point, of 12 ml/g or less, preferably 11 ml/g or less, more preferably 10 ml/g or less, and most preferably 8 ml/g or less.

The oil-absorbing capacity measured at the wet point, noted Wp, corresponds to the amount of oil that needs to be added to 100 g of particles in order to obtain a homogeneous paste. It can be measured according to the wet point method or the method for determining the oil uptake of a powder described in standard NF T 30-022. The oil uptake can correspond to the amount of oil adsorbed onto the available surface of the powder and/or absorbed by the powder by measuring the wet point, described below.

An amount of m = 2 g of powder is placed on a glass plate, and an oil (such as ester oil, oleic acid, or silicone oil) is then added drop-wise. After addition of 4 to 5 drops of oil to the powder, mixing is performed using a spatula, and addition of oil is continued until a conglomerate of oil and powder has formed. At this point, the oil is added one drop at a time and the mixture is then triturated with the spatula. The addition of oil is stopped when a firm, smooth paste is obtained. This paste must be able to be spread on the glass plate without cracking or forming lumps. The volume Vs (expressed in ml) of oil used is then noted. The oil uptake corresponds to the ratio Vs/m.

Otherwise, an oil-absorbing capacity can be measured in accordance with JIS-K6217-4.

In one preferred embodiments of the present invention, the (a) spherical hydrophobic silica aerogels are those described in JP-A-2014-088307, JP-A-2014-218433, or JP-A-2018-177620.

The spherical hydrophobic silica aerogel(s) may be present in an amount of 0.05% by weight or more, preferably 0.1% by weight or more, more preferably 0.2% by weight or more, and even more probably 0.3% by weight or more, and/or may be present in an amount of 5% by weight or less, preferably 4% by weight or less, more preferably 2% by weight or less, and most preferably 1% by weight or less, relative to the total weight of the composition.

The amount of the spherical hydrophobic silica aerogel(s) in the composition according to the present invention may be from 0.05% to 5% by weight, preferably from 0.1% to 4% by weight, more preferably from 0.2% to 2% by weight, and even more preferably 0.3% to 1% by weight, relative to the total weight of the composition.

### Oily Phase

The oily phase of the present invention comprises at least the (b) thickening aid selected from polyoxyalkylenated silicone and alkyl or alkylene carbonate and the (c) film-forming polymer. The oil phase may include any lipophilic, liposoluble or lipodispersible ingredients in addition to the ingredients (b) and (c).

The amount of the oily phase is not particularly limited. In general, the amount of the oily phase may be 5% by weight or more, preferably 10% by weight or more, more preferably 20% by weight or more, and/or may be 55% by weight or less, preferably 45% by weight or less, and more preferably 40% by weight or less relative to the total weight of the composition.

The amount of the oily phase in the composition according to the present invention may be from 5% to 55% by weight, preferably from 10% to 45% by weight, and more preferably from 20% to 40% by weight, relative to the total weight of the composition.

### (Thickening Aid)

The composition according to the present invention comprises at least one thickening aid selected from polyoxyalkylenated silicone and alkyl or alkylene carbonate. Two or more thickening aids may be used in combination. Thus, a single type of the thickening aid or a combination of different types of the thickening aids may be used.

The thickening aid of the present invention is included in the oily phase and is used to aid lipophilic or oil thickener such as the spherical hydrophobic silica aerogel so as to achieve a good stability.

The thickening aid is selected from polyoxyalkylenated silicone and alkyl or alkylene carbonate.

### • Polyoxyalkylenated Silicone

A single type of the polyoxyalkylenated silicone or a combination of different types of the polyoxyalkylenated silicone may be used in the composition according to the present invention.

In the present invention, the term "polyoxyalkylenated silicone" means any silicone comprising at least one oxyalkylenated unit.

The oxyalkylenated units in the polyoxyalkylenated silicone can be polyoxyalkylene groups alternating oxygen atoms and linear or branched C₂ to C₁₀ alkylene groups, preferably C₂ to C₆ alkylene groups, and more preferentially C₂ and/or C₃ (respectively ethylene and propylene) alkylene groups. The oxyalkylenated units can be represented by (-CₓH₂ₓO-)ₐ wherein x ranges from 2 to 6 and a is greater than or equal to 2. Preferably, the oxyalkylenated units are oxyethylene or oxypropylene units, or a combination thereof, and are preferably oxyethylene units.

The polyoxyalkylenated silicone preferably contains a number of moles of ethylene oxide and/or of propylene oxide of between 2 and 100, more preferably between 3 and 50, even more preferably between 4 and 40. In one embodiment of the present invention, the polyoxyalkylenated silicone does not comprise any oxypropylene units.

In one embodiment, the polyoxyalkylenated silicone comprises from 2 to 50 oxyethylene units, preferably from 3 to 40 oxyethylene units, more preferably from 4 to 30 oxyethylene units, and even more preferably from 5 to 20 oxyethylene units.

In one embodiment of the present invention, the polyoxyalkylenated silicone include dimethicone copolyols which are polyorganosiloxanes comprising oxyalkylenated units, such as polyethyleneoxy and/or polypropyleneoxy moieties, preferably a polyethyleneoxy moiety.

The polyoxyalkylenated units of the polyoxyalkylenated silicone can be substituted with at least one alkyl group and/or amine group. The alkyl group may be a linear or branched C₁ to C₃₀ chain, preferably C₄ to C₂₂ chain. The substituted amine moieties may be chosen, for example, from amino C₁-C₄ alkyl moieties.

The polyoxyalkylenated silicone may be a compound of formula (I): 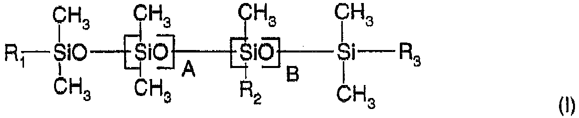 in which:
R₁, R₂ and R₃, independently of each other, represent a C₁-C₆ alkyl radical or a radical - (CH₂)ₓ-(OCH₂CH₂)_{y}-(OCH₂CH₂CH₂)_{z}-OR₄, at least one radical R₁, R₂ or R₃ not being an alkyl radical; R₄ being a hydrogen, an alkyl radical or an acyl radical;
A is an integer ranging from 0 to 200;
B is an integer ranging from 0 to 50; with the proviso that A and B are not simultaneously equal to zero;
x is an integer ranging from 1 to 6;
y is an integer ranging from 1 to 30;
z is an integer ranging from 0 to 5.

According to one embodiment of the present invention, in the compound of formula (I), the alkyl radical is a methyl radical, x is an integer ranging from 2 to 6 and y is an integer ranging from 4 to 30.

As examples of polyoxyalkylenated silicone of formula (I), mention may be made of the compounds of formula (II): 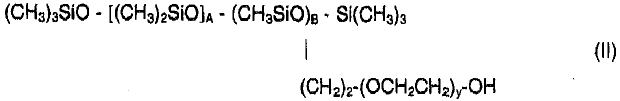 in which A is an integer ranging from 20 to 105, B is an integer ranging from 2 to 10 and y is an integer ranging from 10 to 20.

As examples of the polyoxyalkylenated silicone of formula (I), mention may also be made of the compounds of formula (III):

H-(OCH₂CH₂)_{y}-(CH₂)₃-[(CH₃)₂SiO]_{A'}-(CH₂)₃-(OCH₂CH₂)_{y}-OH (III)

in which A' and y are integers ranging from 10 to 20.

The polyoxyalkylenated silicone of the present invention which may be used are those sold by the company Dow Corning under the names DC 5329, DC 7439-146, DC 2-5695 and Q4-3667. The compounds DC 5329, DC 7439-146 and DC 2-5695 are compounds of formula (III) in which, respectively, A is 22, B is 2 and y is 12; A is 103, B is 10 and y is 12; A is 27, B is 3 and y is 12. The compound Q4-3667 is a compound of formula (III) in which A is 15 and y is 13.

Commercially available products of dimethicone copolyols include, for example, dimethicone copolyolas such as PEG10 dimethicone and PEG14 dimethicone; PEG12 dimethicone marked under the trade name XIAMETER^{®} OFX-0193 FLUID by DOW CORNING, PEG/PPG 18/18 dimethicone silicones sold under the name 5225C by Dow Corning, Cetyl PEG/PPG-10/1 dimethicone silicones such as proposed under the name ABIL EM 90 by Evonik.

### • Alkyl or Alkylene Carbonate

A single type of the alkyl or alkylene carbonate or a combination of different types of the alkyl or alkylene carbonate may be used in the composition according to the present invention.

According to one embodiment, the alkylene chain(s) of the alkylene carbonate(s) and/or the alkyl radical(s) of the alkyl carbonate(s) comprise(s) from 1 to 6 carbon atoms, preferably from 2 to 6 carbon atoms, and more preferably from 2 to 4 carbon atoms, and are eventually substituted by one or more hydroxyl groups.

According to another embodiment, the sum of the carbons of the alkylene chain(s) of alkylene carbonate(s) and/or the sum of the carbons of the alkyl group(s) of the alkyl carbonate(s) present in the composition according to the invention is (are) ranging from 2 to 6 carbon atoms.

The alkylene carbonates are notably chosen from those of formula (4) below: 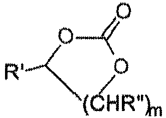 in which formula (4)
R' denotes a hydrogen atom, a linear or branched C₁-C₆ alkyl radical, a linear or branched C₁-C₄ hydroxyalkyl radical;
R" represents a hydrogen atom, a linear or branched C₁-C₆ alkyl radical, a linear or branched C₁-C₄ hydroxyalkyl radical;
m is 1, 2 or 3.

Preferably, the radical R' represents a hydrogen atom, a linear or branched C₁-C₄ alkyl radical, a linear or branched C₁-C₂ hydroxyalkyl radical.

R" represents a hydrogen atom, a linear or branched C₁-C₂ alkyl radical, a linear or branched C₁-C₂ hydroxyalkyl radical.

Preferably, m is 1.

As particularly advantageous examples of alkylene carbonates, mention may be made of the compounds for which the radical R' represents a hydrogen atom (corresponding to ethylene carbonate), a methyl group (corresponding to propylene carbonate), ethyl (corresponding to 1,2-butylene carbonate), hydroxymethyl (R' = -CH₂OH; corresponding to glyceryl carbonate). Preferably, the alkylene carbonate used is propylene carbonate.

The alkyl carbonates are notably chosen from those of formula (5) below:

R' -O-CO-O-R"

in which formula (5)
R' denotes a linear or branched C₁-C₅ alkyl radical, a linear or branched C₁-C₄ hydroxyalkyl radical;
R" represents a linear or branched C₁-C₅ alkyl radical, a linear or branched C₁-C₄ hydroxyalkyl radical;
the sum of the carbons of R' and R" ranging from 2 to 6.

Preferably, the radical R' represents a linear C₁-C₃ alkyl radical or a linear C₁-C₂ hydroxyalkyl radical.

R" represents a linear C₁-C₃ alkyl radical or a linear C₁-C₂ hydroxyalkyl radical.

More particularly, mention may be made of diethyl carbonate and dipropyl carbonate.

The carbonates according to the invention are preferably alkylene carbonates and more particularly propylene carbonate.

The thickening aid(s) may be present in an amount of 0.05% by weight or more, preferably 0.1% by weight or more, more preferably 0.2% by weight or more, and even more probably 0.3% by weight or more, and/or may be present in an amount of 5% by weight or less, preferably 4% by weight or less, more preferably 2% by weight or less, and most preferably 1% by weight or less, relative to the total weight of the composition.

The amount of the thickening aid(s) in the composition according to the present invention may be from 0.05% to 5% by weight, preferably from 0.1% to 4% by weight, more preferably from 0.2% to 2% by weight, and even more preferably 0.3% to 1% by weight relative to the total weight of the composition.

### (Film-Forming Polymer)

The composition according to the present invention comprises at least one film-forming polymer. Two or more film-forming polymers may be used in combination. Thus, a single type of film-forming polymer or a combination of different types of film-forming polymers may be used.

For the purposes of the present invention, the term "polymer" means a compound corresponding to the repetition of one or more units (these units being derived from compounds known as monomers). This or these units(s) are repeated at least twice and preferably at least three times.

The term "film-forming polymer" means a polymer that is capable of forming, by itself or in the presence of an auxiliary film-forming agent, a macroscopically continuous film that adheres to a support, especially to keratin materials, preferably a cohesive film, and better still a film whose cohesion and mechanical properties are such that the said film may be isolable and manipulable in isolation, for example, when the said film is prepared by pouring onto a non-stick surface, for instance, a Teflon-coated or silicone-coated surface.

According to one embodiment of the present invention, the film-forming polymer may be selected from the group comprising:
- film-forming polymers that are soluble in an organic solvent medium, in particular liposoluble polymers; this means that the polymer is soluble or miscible in the organic medium and will form a single homogeneous phase when it is incorporated into the medium;
- film-forming polymers that are dispersible in an organic solvent medium; this means that the polymer forms an insoluble phase in the organic medium, the polymer remaining stable and/or compatible once incorporated into this medium. In particular, such polymers may be in the form of non-aqueous dispersions of polymer particles, preferably dispersions in silicone-based or hydrocarbon-based oils; in one embodiment, the non-aqueous dispersions of polymer comprise polymer particles stabilized on their surface with at least one stabilizer; these non-aqueous dispersions are often referred to as "NADs"; and
- film-forming polymers in the form of aqueous dispersions of polymer particles; this means that the polymer forms an insoluble phase in water, the polymer remaining stable and/or compatible once incorporated into the water, the polymer particles possibly being stabilized at their surface with at least one stabilizer. These polymer particles are often referred to as "lattices"; in this case, the composition must comprise an aqueous phase.

Preferably, the film-forming polymer is selected from the group consisting of polyamide-silicone block polymers, block ethylenic polymers, vinyl polymers comprising at least one carboxiloxane dendrimer derivative, copolymers comprising carboxylate groups and polydimethylsilixane groups, silicone resins, lipodispersible polymers in the form of a non-aqueous dispersion of polymer particles, olefin copolymers selected from amorphous olefin copolymers and olefin copolymers with controlled and moderate crystallization, hydrocarbon-based resins having a number-average molecular weight of less than or equal to 10,000 g/ml, and a mixture thereof, more preferably from silicone resins.

The film-forming silicone resin may be any silicone resin which has film-forming properties.

According to one embodiment of the present invention, the film-forming silicone resin may be selected from silsesquioxane, siloxysilicate and a resin obtained by hydroxysilylation.

The nomenclature of the silicone resin is known in the art under the name of "MDTQ" nomenclature, by which a silicone resin is described according to the various repeating siloxane monomer moieties which constitute the polymer. Each letter of "MDTQ" corresponds to a different type of moiety.

The symbol "M" corresponds to the monofunctional moiety (CH₃)₃SiO_{1/2}. This moiety is regarded as monofunctional because the silicon atom shares only one oxygen for the formation of the chain. The "M" moiety can be represented by the following structure:

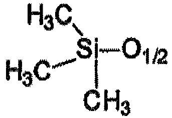

At least one of the methyl groups can be replaced so as, for example, to produce a moiety with the following formula: [R(CH₃)₂] SiO_{1/2}, such as represented by the following structure: 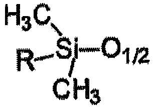 in which R is other than a methyl group.

The symbol "D" corresponds to the difunctional moiety (CH₃)SiO_{2/2} in which two of the available bonds on the silicon atom are used to bond with oxygen for the formation of the polymer chain. The "D" moiety, which is the essential component element of the dimethicone oils, can be represented by the following formula:

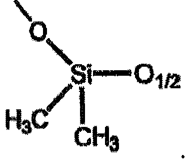

The symbol "T" corresponds to the trifunctional moiety (CH₃)SiO_{3/2}, in which three of the available bonds on the silicon atom are used to bond with oxygen for the formation of the polymer chain. The "T" moiety can be represented by the following structure:

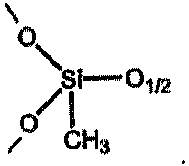

As in the "M" moiety, any one of the methyl groups can be replaced in "D" or "T" by an R group which is other than methyl.

Finally, the symbol "Q" corresponds to a quadrifunctional moiety SiO_{4/2}, in which all four available bonds on the silicon atom are used to bond with oxygen for the formation of the polymer chain. The "Q" moiety can be represented by the following structure:

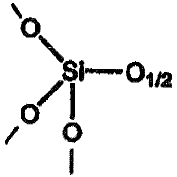

As described above, in one embodiment of the present invention, the film-forming silicone resin may be selected from the siloxysilicate, silsesquioxane and a resin obtained by hydroxysililation. Any siloxysilicate, silsesquioxane or resin obtained by hydroxysilylation, which acts as a film-forming agent, can be used in the composition of the present invention. The film-forming silicone resin preferably is crosslinked.

According to one embodiment of the present invention, the film-forming silicone resin may be selected from substituted siloxysilicate, silsesquioxane and resin obtained by hydroxysilylation. A substituted siloxysilicate or a substituted silsesquioxane may be, for example, a siloxysilicate or a silsesquioxane in which a methyl group has been replaced by a longer carbon chain, such as an ethane, propane or butane chain. The carbon chain may be saturated or nonsaturated.

According to one embodiment of the present invention, the film-forming silicone resin may be selected from siloxysilicate, such as MQ resins represented by the following formula:

[(CH₃)₃SiO_{1/2}]ₓ(SiO_{4/2})_{y} (MQ moieties)

in which x and y may have values ranging from 20 to 100, preferably 50 to 80.

According to another embodiment of the present invention, siloxysilicate may be selected from all the combinations of M and of Q moieties such as, for example, [(R)₃Si]ₓ(SiO_{4/2})_{y}, in which R is selected from a methyl group and a longer carbon chain, such as C₂-C₁₀ alkyl groups.

According to another embodiment of the present invention, the film-forming silicone resin may be selected from silsesquioxane represented by the following formula:

(CH₃SiO_{3/2})ₓ (T moieties),

in which x has a value which can range up to several thousands and the CH₃ can be replaced by an R, such as described hereinabove for the T moieties.

Most preferably, the film-forming silicone resin is trimethylsiloxysilicate, for example, sold by the company Momentive Performance Materials under the name SR 1000 MQ Resin.

The film-forming polymer(s) may be present in an amount of 0.5% by weight or more, preferably 1% by weight or more, more preferably 3% by weight or more, and even more probably 5% by weight or more, and/or may be present in an amount of 20% by weight or less, preferably 15% by weight or less, more preferably 10% by weight or less, and most preferably 8% by weight or less, relative to the total weight of the composition.

The amount of the film-forming polymer(s) in the composition according to the present invention may be from 0.5% to 20% by weight, preferably from 1% to 15% by weight, more preferably from 3% to 10% by weight, and even more preferably 5% to 8% by weight relative to the total weight of the composition.

### Aqueous Phase

The aqueous phase of the present invention comprises at least the (d) polyol and the (e) anionic polymer. The aqueous phase may include an aqueous medium, i.e., water and optionally a water-soluble solvent in addition to the ingredients (d) and (e).

The amount of the aqueous phase is at least 40% by weight relative to the total weight of the composition. The amount of the aqueous phase may be 90% by weight or less, preferably 80% by weight or less, more preferably 70% by weight or less, and even more preferably 60% by weight or less, relative to the total weight of the composition.

In the present invention, the term "water-soluble solvent" denotes a compound that is liquid at room temperature and water-miscible (miscibility with water of greater than 50% by weight at 25°C and atmospheric pressure).

### (Polyol)

The composition according to the present invention comprises at least one polyol. Two or more polyols may be used in combination. Thus, a single type of the polyol or a combination of different types of the polyols may be used.

For the purposes of the present invention, the term "polyol" should be understood as meaning any organic molecule comprising at least two free hydroxyl groups.

The polyol that is suitable for use in the invention may be a compound of linear, branched or cyclic, saturated or unsaturated alkyl type, bearing at least two -OH functions on the alkyl chain.

Preferably, a polyol that may be used in the composition according to the invention is a compound of linear or branched, preferably linear alkyl type bearing at least two -OH functions, preferably 2 to 5 -OH functions, more preferably 2 to 4 -OH functions, and even more preferably 2 or 3 -OH functions on the alkyl chain.

The polyols that are advantageously suitable for formulating the cosmetic compositions according to the present invention are those especially having from 2 to 8 carbon atoms or, for example 3 to 6 carbon atoms.

The polyols that may be used according to the present invention are chosen from linear or branched, preferably linear polyols having from 3 to 8 carbon atoms; mention may be made especially of:
- diols such as hexylene glycol, dipropylene glycol, pentylene glycol, propylene glycol and butylene glycol; and
- triols, such as glycerol (glycerin),
and mixtures thereof.

The amount of polyol(s) in the composition is at least 6% by weight relative to the total weight of the composition. The polyol(s) may be present in an amount of 30% by weight or less, preferably 25% by weight or less, more preferably 20% by weight or less, and even more preferably 15% by weight or less, relative to the total weight of the composition.

In one preferred embodiment of the present invention, the polyol comprises at least one diol and at least one polyol having three or more -OH functions, in particular triol, in combination. In this embodiment, the composition according to the present invention may comprise at least 3% by weight of the diol and at least 3% by weight of the polyol having three or more -OH functions in combination, relative to the total weight of the composition.

Specifically, the composition may comprise a combination of at least one diol and at least one triol. In this embodiment, the composition according to the present invention may comprise at least 3% by weight of the diol and at least 3% by weight of the triol in combination, relative to the total weight of the composition. In another embodiment, the composition according to the present invention comprises the diol in an amount range from 1% to 20% by weight and the triol in an amount ranging from 1% to 10% by weight; more preferably the diol in an amount ranging from 2% to 15% by weight and the triol in an amount ranging from 2% to 10% by weight; and even more preferably the diol in an amount ranging from 3% to 15% by weight and the triol in an amount ranging from 3% to 10% by weight relative to the total weight of the composition.

### (Anionic Polymer)

The composition according to the present invention comprises at least one anionic polymer. Two or more types of anionic polymer may be used in combination. Thus, a single type of the anionic polymer or a combination of different types of the anionic polymer may be used.

The anionic polymer is present in the aqueous phase of the present invention. Therefore, the anionic polymer here can be water-soluble, and hydrophilic. For the purpose of the present invention, the term "hydrophilic" here means that a substance is soluble in water at a concentration of at least 1% by weight relative to the total weight of the water at room temperature (25°C) and atmospheric pressure (10⁵ Pa).

An anionic polymer has a positive charge density. The charge density of the anionic polymer may be from 0.1 meq/g to 20 meq/g, preferably from 1 to 15 meq/g, and more preferably from 4 to 10 meq/g if the anionic polymer is a synthetic anionic polymer, and the average substitution degree of the anionic polymer may be from 0.1 to 3.0, preferably from 0.2 to 2.7, and more preferably from 0.3 to 2.5 if the anionic polymer is a natural anionic polymer.

It may be preferable that the molecular weight of the anionic polymer be 1,000 or more, preferably 5,000 or more, even more preferably 10,000 or more, even more preferably 15,000 or more, and in particular 20,000 or more; and/or be 1,000,000 or less, preferably 500,000 or less, more preferably 200,000 or less, and even more preferably 100,000 or less, an in particular 50,000 or less.

It is prefered that the molecular weight of the anionic polymer be 1,000 or more, preferably 3,000 or more, and/or be 1,000,000 or less, preferably 500,000 or less, more preferably 200,000 or less, and even more preferably 100,000 or less, an in particular 50,000 or less.

In one preferred embodiment, the molecular weight of the anionic polymer ranges from 1,000 to 1,000,000, preferably from 2,000 to 500,000, even more preferably from 3,000 to 50,000.

Unless otherwise defined in the descriptions, "molecular weight" may mean a number average molecular weight.

The anionic polymer may have at least one negatively chargeable and/or negatively charged moiety selected from the group consisting of a sulfuric group, a sulfate group, a sulfonic group, a sulfonate group, a phosphoric group, a phosphate group, a phosphonic group, a phosphonate group, a carboxylic group, and a carboxylate group.

The anionic polymer may be a homopolymer or a copolymer. The term "copolymer" is understood to mean both copolymers obtained from two kinds of monomers and those obtained from more than two kinds of monomers, such as terpolymers obtained from three kinds of monomers.

The anionic polymer may be selected from natural and synthetic anionic polymers.

The anionic polymer may comprise at least one hydrophobic chain.

The anionic polymer which may comprise at least one hydrophobic chain may be obtained by copolymerization of a monomer (a) chosen from carboxylic acids comprising an α,β-ethylenic unsaturation (monomer a') and 2-acrylamido-2-methylpropanesulphonic acid (monomer a") with a non-surface-active monomer (b) comprising an ethylenic unsaturation other than (a) and/or a monomer (c) comprising an ethylenic unsaturation resulting from the reaction of an acrylic monomer comprising an α,β-monoethylenic unsaturation or of an isocyanate monomer comprising a monoethylenic unsaturation with a monohydric nonionic amphiphilic component or with a primary or secondary fatty amine.

Thus, the anionic polymer with at least one hydrophobic chain may be obtained by two synthetic routes:
- either by copolymerization of the monomers (a') and (c), or (a'), (b) and (c), or (a") and (c), or (a"), (b) and (c),
- or by modification (and in particular esterification or amidation) of a copolymer formed from the monomers (a') or from the monomers (a') and (b), or (a") and (b), by a monohydric nonionic amphiphilic compound or a primary or secondary fatty amine.

Mention may in particular be made, as 2-acrylamido-2-methylpropanesulphonic acid copolymers, of those disclosed in the article "Micelle formation of random copolymers of sodium 2-(acrylamido)-2-methylpropanesulfonate and nonionic surfactant macromonomer in water as studied by fluorescence and dynamic light scattering - Macromolecules, 2000, Vol. 33, No. 10 - 3694-3704" and in applications EP-A-0 750 899 and EP-A-1 069 172.

The carboxylic acid comprising an α,β-monoethylenic unsaturation constituting the monomer (a') can be chosen from numerous acids and in particular from acrylic acid, methacrylic acid, crotonic acid, itaconic acid and maleic acid. It is preferably acrylic or methacrylic acid.

The copolymer can comprise a monomer (b) comprising a monoethylenic unsaturation which does not have a surfactant property. The preferred monomers are those which give water-insoluble polymers when they are homopolymerized. They can be chosen, for example, from C₁-C₄ alkyl acrylates and methacrylates, such as methyl acrylate, ethyl acrylate, butyl acrylate or the corresponding methacrylates. The more particularly preferred monomers are methyl acrylate and ethyl acrylate. The other monomers which can be used are, for example, styrene, vinyltoluene, vinyl acetate, acrylonitrile and vinylidene chloride. Unreactive monomers are preferred, these monomers being those in which the single ethylenic group is the only group which is reactive under the polymerization conditions. However, monomers which comprise groups which react under the effect of heat, such as hydroxyethyl acrylate, can optionally be used.

The monomer (c) is obtained by reaction of an acrylic monomer comprising α,β-monoethylenic unsaturation, such as (a), or of an isocyanate monomer comprising monoethylenic unsaturation with a monohydric nonionic amphiphilic compound or a primary or secondary fatty amine.

The monohydric nonionic amphiphilic compounds or the primary or secondary fatty amines used to produce the nonionic monomer (c) are well known. The monohydric nonionic amphiphilic compounds are generally alkoxylated hydrophobic compounds comprising an alkylene oxide forming the hydrophilic part of the molecule. The hydrophobic compounds are generally composed of an aliphatic alcohol or an alkylphenol, in which compounds a carbonaceous chain comprising at least six carbon atoms constitutes the hydrophobic part of the amphiphilic compound.

The preferred monohydric nonionic amphiphilic compounds are compounds having the following formula (V):

R-(OCH₂CHR')ₘ-(OCH₂CH₂)ₙ-OH (V)

in which R is chosen from alkyl or alkylene groups comprising from 6 to 30 carbon atoms and alkylaryl groups having alkyl radicals comprising from 8 to 30 carbon atoms, R' is chosen from alkyl groups comprising from 1 to 4 carbon atoms, n is a mean number ranging from approximately 1 to 150 and m is a mean number ranging from approximately 0 to 50, provided that n is at least as great as m.

Preferably, in the compounds of formula (V), the R group is chosen from alkyl groups comprising from 12 to 26 carbon atoms and alkylphenyl groups in which the alkyl group is C₈-C₁₃; the R' group is the methyl group; m = 0 and n = 1 to 25.

The preferred primary and secondary fatty amines are composed of one or two alkyl chains comprising from 6 to 30 carbon atoms.

The monomer used to form the nonionic urethane monomer (c) can be chosen from highly varied compounds. Use may be made of any compound comprising a copolymerizable unsaturation, such as an acrylic, methacrylic or allylic unsaturation. The monomer (c) can be obtained in particular from an isocyanate comprising a monoethylenic unsaturation, such as, in particular, α,α-dimethyl-m-isopropenylbenzyl isocyanate.

The monomer (c) can be chosen in particular from acrylates, methacrylates or itaconates of oxyethylenated (1 to 50 EO) C₆-C₃₀ fatty alcohol, such as steareth-20 methacrylate, oxyethylenated (25 EO) behenyl methacrylate, oxyethylenated (20 EO) monocetyl itaconate, oxyethylenated (20 EO) monostearyl itaconate or the acrylate modified by polyoxyethylenated (25 EO) C₁₂-C₂₄ alcohols and from dimethyl-m-isopropenylbenzyl isocyanates of oxyethylenated (1 to 50 EO) C₆-C₃₀ fatty alcohol, such as, in particular, the dimethyl-m-isopropenylbenzyl isocyanate of oxyethylenated behenyl alcohol.

According to a specific embodiment of the present invention, the anionic polymer is chosen from acrylic terpolymers obtained from (a) a carboxylic acid comprising an α,β-ethylenic unsaturation, (b) a non-surface-active monomer comprising an ethylenic unsaturation other than (a), and (c) a nonionic urethane monomer which is the reaction product of a monohydric nonionic amphiphilic compound with an isocyanate comprising a monoethylenic unsaturation.

Mention may in particular be made, as anionic polymers comprising at least one hydrophobic chain, of the acrylic acid/ethyl acrylate/alkyl acrylate terpolymer, such as the product as a 30% aqueous dispersion sold under the name Acusol 823 by Rohm & Haas; the acrylates/steareth-20 methacrylate copolymer, such as the product sold under the name Aculyn 22 by Rohm & Haas; the (meth)acrylic acid/ethyl acrylate/oxyethylenated (25 EO) behenyl methacrylate terpolymer, such as the product as an aqueous emulsion sold under the name Aculyn 28 by Rohm & Haas; the acrylic acid/oxyethylenated (20 EO) monocetyl itaconate copolymer, such as the product as a 30% aqueous dispersion sold under the name Structure 3001 by National Starch; the acrylic acid/oxyethylenated (20 EO) monostearyl itaconate copolymer, such as the product as a 30% aqueous dispersion sold under the name Structure 2001 by National Starch; the acrylates/acrylate modified by polyoxyethylenated (25 EO) C₁₂-C₂₄ alcohol copolymer, such as the 30-32% copolymer latex sold under the name Synthalen W2000 by 3V SA; or the methacrylic acid/methyl acrylate/dimethyl-meta-isopropenylbenzyl isocyanate of ethoxylated behenyl alcohol terpolymer, such as the product as a 24% aqueous dispersion and comprising 40 ethylene oxide groups disclosed in the document EP-A-0 173 109.

The anionic polymers may also be Polyester-5, such as the product sold under the name of Eastman AQ^{™} 55S Polymer by EASTMAN CHEMICAL having the chemical formula below.

HO-G-A-G-A-G-A-G-A-G-A-G-A-G-A-G-A-G-OH

SO₃-Na+ SO₃⁻Na⁺

A: dicarboxylic acid moiety
G: glycol moiety
SO₃⁻Na⁺: sodium sulfo group
OH: hydroxyl group

It may be preferable that the anionic polymer be selected from the group consisting of polysaccharides such as alginic acid, hyaluronic acid, and cellulose polymers (e.g., carboxymethylcellulose), anionic (co)polyaminoacids such as (co)polyglutamic acids, (co)poly(meth)acrylic acids, (co)polyamic acids, (co)polystyrene sulfonate, (co)poly(vinyl sulfate), dextran sulfate, chondroitin sulfate, (co)polymaleic acids, (co)polyfumaric acids, maleic acid (co)polymers, and salts thereof.

The maleic acid copolymer may comprise one or more maleic acid comonomers, and one or more comonomers chosen from vinyl acetate, vinyl alcohol, vinylpyrrolidone, olefins comprising from 2 to 20 carbon atoms, and styrene.

Thus, the "maleic acid copolymer" is understood to mean any polymer obtained by copolymerization of one or more maleic acid comonomers and of one or more comonomers chosen from vinyl acetate, vinyl alcohol, vinylpyrrolidone, olefins comprising from 2 to 20 carbon atoms, such as octadecene, ethylene, isobutylene, diisobutylene or isooctylene, and styrene, the maleic acid comonomers optionally being partially or completely hydrolysed. Use will preferably be made of hydrophilic polymers, that is to say polymers having a solubility in water of greater than or equal to 2 g/l.

In an advantageous aspect of the present invention, the maleic acid copolymer may have a molar fraction of maleic acid units of between 0.1 and 1, more preferably between 0.4 and 0.9.

The weight-average molar mass of the maleic acid copolymer may be between 1,000 and 500,000, and preferably between 1,000 and 50,000.

It is preferable that the maleic acid copolymer be a styrene/maleic acid copolymer, and more preferably sodium styrene/maleic acid copolymer.

Use will preferably be made of a copolymer of styrene and of maleic acid in a 50/50 ratio.

Use may be made, for example, of the styrene/maleic acid (50/50) copolymer, in the form of an ammonium salt at 30% in water, sold under the reference SMA1000H^{®} by Cray Valley or the styrene/maleic acid (50/50) copolymer, in the form of a sodium salt at 40% in water, sold under the reference SMA1000HNa^{®} by Cray Valley.

The use of the styrene/maleic acid copolymer such as sodium styrene/maleic acid copolymer may improve the wettability of a film prepared by the composition according to the present invention.

According to one embodiment of the present invention, it is preferable that the anionic polymer be selected from hyaluronic acid and derivatives thereof.

Hyaluronic acid can be represented by the following chemical formula.

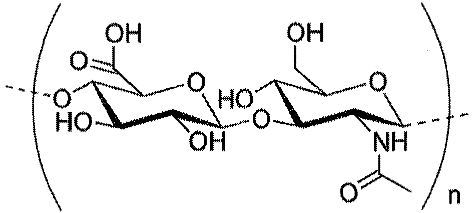

In the context of the present invention, the term "hyaluronic acid" covers in particular the basic unit of hyaluronic acid of formula:

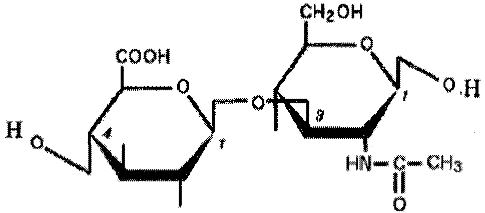

This is the smallest fraction of hyaluronic acid comprising a disaccharide dimer, namely D-glucuronic acid and N-acetylglucosamine.

The term "hyaluronic acid and derivatives thereof" also comprises, in the context of the present invention, the linear polymer comprising the polymeric unit described above, linked together in the chain via alternating β(1,4) and β(1,3) glycosidic linkages, having a molecular weight (MW) that can range between 380 and 1,000,000 daltons. This molecular weight depends in large part on the source from which the hyaluronic acid is obtained and/or on the preparation methods.

The term "hyaluronic acid and derivatives thereof" also comprises, in the context of the present invention, hyaluronic acid salts. As the salts, mention may be made of alkaline metal salts such as sodium salts and potassium salts, alkaline earth metal salts such as magnesium salts, ammonium salts, and mixtures thereof.

In the natural state, hyaluronic acid is present in pericellular gels, in the base substance of the connective tissues of vertebrate organs such as the dermis and epithelial tissues, and in particular in the epidermis, in the synovial fluid of the joints, in the vitreous humor, in the human umbilical cord and in the crista galli apophysis.

Thus, the term "hyaluronic acid and derivatives thereof" comprises all the fractions or subunits of hyaluronic acid having a molecular weight in particular within the molecular weight range recalled above.

In the context of the present invention, hyaluronic acid fractions which do not have inflammatory activity are preferably used.

By way of illustration of the various hyaluronic acid fractions, reference may be made to the document "Hyaluronan fragments: an information-rich system", R. Stern et al., European Journal of Cell Biology 58 (2006) 699-715, which reviews the listed biological activities of hyaluronic acid according to its molecular weight.

According to a preferred embodiment of the present invention, the hyaluronic acid fractions suitable for the use covered by the present invention have a molecular weight of less than 50,000 Da, which is a so-called low-molecular-weight hyaluronic acid.

Finally, the term "hyaluronic acid and derivatives thereof" also comprises hyaluronic acid esters in particular those in which all or some of the carboxylic groups of the acid functions are esterified with oxyethylenated alkyls or alcohols, containing from 1 to 20 carbon atoms, in particular with a degree of substitution at the level of the D-glucuronic acid of the hyaluronic acid ranging from 0.5 to 50%.

Mention may in particular be made of methyl, ethyl, n-propyl, n-pentyl, benzyl and dodecyl esters of hyaluronic acid. Such esters have in particular been described in D. Campoccia et al. "Semisynthetic resorbable materials from hyaluronan esterification", Biomaterials 19 (1998) 2101-2127.

The hyaluronic acid derivative may be, for example, acetylated hyaluronic acid or a salt thereof.

The molecular weights indicated above are also valid for the hyaluronic acid esters.

The amount of the anionic polymer(s) in the composition according to the present invention may be 0.01% by weight or more, preferably 0.05% by weight or more, and more preferably 0.1% by weight or more, relative to the total weight of the composition.

The amount of the anionic polymer(s) in the composition according to the present invention may be 20% by weight or less, preferably 15% by weight or less, and more preferably 10% by weight or less, relative to the total weight of the composition.

The amount of the anionic polymer(s) in the composition according to the present invention may be from 0.01% to 20% by weight, preferably from 0.05% to 15% by weight, and more preferably from 0.1% to 10% by weight, relative to the total weight of the composition.

### (Other Ingredients)

### • Oil

The composition according to the present invention may comprise at least one oil in the oily phase. Two or more types of oils may be used in combination. Thus, a single type of oil or a combination of different types of oils may be used.

Here, "oil" means a fatty compound or substance which is in the form of a liquid or a paste (non-solid) at room temperature (25 °C) under atmospheric pressure (760 mmHg). As the oils, those generally used in cosmetics can be used alone or in combination. These oils may be volatile or non-volatile.

Among the oils which may be used in the present invention, mention may be made of: volatile or non-volatile oils; these oils may be hydrocarbon-based oils, especially of animal or plant origin, synthetic oils, silicone oils, fluoro oils, or mixtures thereof.

For the purposes of the present invention, "hydrocarbon-based oil" or "hydrocarbon oil" is intended to mean an oil mainly containing hydrogen and carbon atoms and optionally oxygen, nitrogen, sulfur and/or phosphorus atoms. The hydrocarbon-based oil does not comprise any silicon atoms.

For the purposes of the present invention, "silicone oil" is intended to mean an oil comprising at least one silicon atom, and especially at least one Si-O group.

For the purposes of the present invention, "polar oil" is intended to mean an oil of which the solubility parameter δₐ at 25°C is other than 0 (J/cm³)^{1/2}.

In particular, "polar oil" is intended to mean an oil of which the chemical structure is formed essentially from, or even constituted of, carbon and hydrogen atoms, and comprising at least one highly electronegative heteroatom such as an oxygen, nitrogen, silicon or phosphorus atom.

The definition and calculation of the solubility parameters in the Hansen three-dimensional solubility space are described in the article by C.M. Hansen: The three-dimensional solubility parameters, J. Paint Technol., 39, 105 (1967).

According to this Hansen space:
- δ_{D} characterizes the London dispersion forces resulting from the formation of dipoles induced during molecular impacts;
- δₚ characterizes the Debye interaction forces between permanent dipoles and also the Keesom interaction forces between induced dipoles and permanent dipoles;
- δₕ characterizes the forces of specific interactions (such as hydrogen bonds, acid/base bonds, donor/acceptor bonds, and the like);
- δₐ is determined by the equation: ${δ}_{a} = {\left({{δ}_{p}}^{2}+{{δ}_{h}}^{2}\right)}^{\frac{1}{2}} .$

The parameters δₚ, δₕ, δ_{D} and δₐ are expressed as (J/cm³)^{½}.

Preferably, the polar oils used according to the present invention have a δₐ of between 4 and 9.1, preferably a δₐ of between 6 and 9.1, even better still between 7.3 and 9.1.

The oil may be a non-polar oil such as a hydrocarbon oil, a silicone oil, or the like; a polar oil such as a plant or animal oil and an ester oil or an ether oil; or a mixture thereof.

The oil may be selected from the group consisting of oils of plant or animal origin, synthetic oils, silicone oils, hydrocarbon oils, and fatty alcohols.

As examples of plant oils, mention may be made of, for example, linseed oil, camellia oil, macadamia nut oil, corn oil, mink oil, olive oil, avocado oil, sasanqua oil, castor oil, safflower oil, jojoba oil, sunflower oil, almond oil, rapeseed oil, sesame oil, soybean oil, peanut oil, and mixtures thereof.

As examples of animal oils, mention may be made of, for example, squalene and squalane.

As examples of synthetic oils, mention may be made of alkane oils such as isododecane and isohexadecane, ester oils apart from the alkyl or alkylene carbonate, ether oils, and artificial triglycerides.

The ester oils are preferably liquid esters of saturated or unsaturated, linear or branched C₁-C₂₆ aliphatic monoacids or polyacids and of saturated or unsaturated, linear or branched C₁-C₂₆ aliphatic monoalcohols or polyalcohols, the total number of carbon atoms of the esters being greater than or equal to 10.

Preferably, for the esters of monoalcohols, at least one from among the alcohol and the acid, from which the esters of the present invention are derived, is branched.

The ester oils of the monoesters of monoacids and of monoalcohols may be represented by formula R₁COOR₂ in which R₁ represents the residue of a linear or branched, preferably a linear fatty acid comprising from 1 to 40 carbon atoms, preferably 6 to 24 carbon atoms, and more preferably 10 to 20 carbon atoms, and R₂ represents a hydrocarbon-based chain, especially branched, containing from 1 to 40 carbon atoms, preferably 1 to 12 carbon atoms, and more preferable 2 to 8 carbon atoms, with the proviso that R₁ + R₂ is ≥ 10.

Among the monoesters of monoacids and of monoalcohols, mention may be made of ethyl palmitate, ethyl hexyl palmitate, isopropyl palmitate, alkyl myristates such as isopropyl myristate or ethyl myristate, isocetyl stearate, 2-ethylhexyl isononanoate, isononyl isononanoate, isodecyl neopentanoate, and isostearyl neopentanoate.

It is preferred that the ester oil is selected from fatty acid ester oils.

Esters of C₄-C₂₂ dicarboxylic or tricarboxylic acids and of C₁-C₂₂ alcohols, and esters of monocarboxylic, dicarboxylic, or tricarboxylic acids and of non-sugar C₄-C₂₆ dihydroxy, trihydroxy, tetrahydroxy, or pentahydroxy alcohols may also be used.

Mention may especially be made of: diethyl sebacate; diisopropyl sebacate; bis(2-ethylhexyl) sebacate; diisopropyl adipate; di-n-propyl adipate; dioctyl adipate; bis(2-ethylhexyl) adipate; diisostearyl adipate; bis(2-ethylhexyl) maleate; triisopropyl citrate; triisocetyl citrate; triisostearyl citrate; glyceryl trilactate; glyceryl trioctanoate; trioctyldodecyl citrate; trioleyl citrate; neopentyl glycol diheptanoate; diethylene glycol diisononanoate.

As ester oils, one can use sugar esters and diesters of C₆-C₃₀ and preferably C₁₂-C₂₂ fatty acids. It is recalled that the term "sugar" means oxygen-bearing hydrocarbon-based compounds containing several alcohol functions, with or without aldehyde or ketone functions, and which comprise at least 4 carbon atoms. These sugars may be monosaccharides, oligosaccharides, or polysaccharides.

Examples of suitable sugars that may be mentioned include sucrose (or saccharose), glucose, galactose, ribose, fucose, maltose, fructose, mannose, arabinose, xylose, and lactose, and derivatives thereof, especially alkyl derivatives, such as methyl derivatives, for instance, methylglucose.

The sugar esters of fatty acids may be chosen especially from the group comprising the esters or mixtures of esters of sugars described previously and of linear or branched, saturated or unsaturated C₆-C₃₀ and preferably C₁₂-C₂₂ fatty acids. If they are unsaturated, these compounds may have one to three conjugated or non-conjugated carbon-carbon double bonds.

The esters according to this variant may also be selected from monoesters, diesters, triesters, tetraesters, and polyesters, and mixtures thereof.

These esters may be, for example, oleates, laurates, palmitates, myristates, behenates, cocoates, stearates, linoleates, linolenates, caprates, and arachidonates, or mixtures thereof such as, especially, oleopalmitate, oleostearate, and palmitostearate mixed esters, as well as pentaerythrityl tetraethyl hexanoate.

More particularly, use is made of monoesters and diesters and especially sucrose, glucose, or methylglucose monooleates or dioleates, stearates, behenates, oleopalmitates, linoleates, linolenates, and oleostearates.

An example that may be mentioned is the product sold under the name Glucate^{®} DO by the company Amerchol, which is a methylglucose dioleate.

As examples of preferable ester oils, mention may be made of, for example, diisopropyl adipate, dioctyl adipate, 2-ethylhexyl hexanoate, ethyl laurate, cetyl octanoate, octyldodecyl octanoate, isodecyl neopentanoate, myristyl propionate, 2-ethylhexyl 2-ethylhexanoate, 2-ethylhexyl octanoate, 2-ethylhexyl caprylate/caprate, methyl palmitate, ethyl palmitate, isopropyl palmitate, isononyl isononanoate, ethylhexyl palmitate, isohexyl laurate, hexyl laurate, isocetyl stearate, isopropyl isostearate, isopropyl myristate, isodecyl oleate, glyceryl tri(2-ethylhexanoate), pentaerythrithyl tetra(2-ethylhexanoate), 2-ethylhexyl succinate, diethyl sebacate, and mixtures thereof.

As the ether oil, dialkyl ethers such as those represented by the following formula:

R¹-O-R²

wherein
each of R¹ and R² independently denotes a linear, branched or cyclic *C*₄*-C*₂₄ alkyl group, preferably C₆-C₁₈ alkyl group, and more preferably C₈-C₁₂ alkyl group. It is preferable that R¹ and R² are the same.

As the linear alkyl group, mention may be made of a butyl group, a pentyl group, a hexyl group, a heptyl group, an octyl group, a nonyl group, a decyl group, an undecyl group, a dodecyl group, tridecyl group, a tetradecyl group, a pentadecyl group, a hexadecyl group, a heptadecyl group, an octadecyl group, a nonadecyl group, an eicosyl group, a behenyl group, a docosyl group, a tricosyl group, and a tetracosyl group.

As the branched alkyl group, mention may be made of a 1-methylpropyl group, 2-methylpropyl group, a t-butyl group, a 1,1-dimethylpropyl group, a 3-methylhexyl group, a 5-methylhexyl group, an 1-ethylhexyl group, an 2-ethylhexylgroup, a 1-butylpentyl group, a 5-methyloctyl group, an 1-ethylhexyl group, an 2-ethylhexyl group, a 1-butylpentyl group, a 5-methyloctyl group, a 2-butyloctyl group, an isotridecyl group, a 2-pentylnonyl group, a 2-hexyldecyl group, an isostearyl group, a 2-heptylundecyl group, an 2-octyldodecyl group, a 1,3-dimethylbutyl group, a 1-(1-methylethyl)-2-methylpropyl group, a 1,1,3,3-tetramethylbutyl group, a 3,5,5-trimethylhexyl group, a 1-(2-methylpropyl)-3-methylbutyl group, a 3,7-dimethyloctyyl group, and a 2-(1,3,3-trimethylbutyl)-5,7,7-trimethyloctyl group.

As the cyclic alkyl group, mention may be made of a cyclohexyl group, a 3-methylcyclohexyl group, and a 3,3,5-trimethylcyclohexyl group.

As examples of artificial triglycerides, mention may be made of, for example, capryl caprylyl glycerides, glyceryl trimyristate, glyceryl tripalmitate, glyceryl trilinolenate, glyceryl trilaurate, glyceryl tricaprate, glyceryl tricaprylate, glyceryl tri(caprate/caprylate), and glyceryl tri(caprate/caprylate/linolenate).

As examples of silicone oils, mention may be made of, for example, linear organopolysiloxanes such as dimethylpolysiloxane, methylphenylpolysiloxane, methylhydrogenpolysiloxane, and the like; cyclic organopolysiloxanes such as cyclohexasiloxane, octamethylcyclotetrasiloxane, decamethylcyclopentasiloxane, dodecamethylcyclohexasiloxane, and the like; and mixtures thereof.

Preferably, the silicone oil is chosen from liquid polydialkylsiloxanes or dimethicone, especially liquid polydimethylsiloxanes (PDMS) and liquid polyorganosiloxanes comprising at least one aryl group.

These silicone oils may also be organomodified. The organomodified silicones that can be used in accordance with the present invention are silicone oils as defined above and comprise in their structure one or more organofunctional groups attached via a hydrocarbon-based group.

Organopolysiloxanes are defined in greater detail in Walter Noll's Chemistry and Technology of Silicones (1968), Academic Press. They may be volatile or non-volatile.

When they are volatile, the silicones are more particularly chosen from those having a boiling point of between 60°C and 260°C, and even more particularly from:
(i) cyclic polydialkylsiloxanes comprising from 3 to 7 and preferably 4 to 5 silicon atoms. These are, for example, octamethylcyclotetrasiloxane sold in particular under the name Volatile Silicone^{®} 7207 by Union Carbide or Silbione^{®} 70045 V2 by Rhodia, decamethylcyclopentasiloxane sold under the name Volatile Silicone^{®} 7158 by Union Carbide, Silbione^{®} 70045 V5 by Rhodia, and dodecamethylcyclopentasiloxane sold under the name Silsoft 1217 by Momentive Performance Materials, and mixtures thereof. Mention may also be made of cyclocopolymers of the type such as dimethylsiloxane/methylalkylsiloxane, such as Silicone Volatile^{®} FZ 3109 sold by the company Union Carbide, of the formula:
   with D": 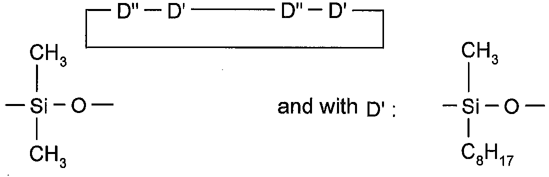 Mention may also be made of mixtures of cyclic polydialkylsiloxanes with organosilicon compounds, such as the mixture of octamethylcyclotetrasiloxane and tetratrimethylsilylpentaerythritol (50/50) and the mixture of octamethylcyclotetrasiloxane and oxy-1,1'-bis(2,2,2',2',3,3'-hexatrimethylsilyloxy)neopentane; and
(ii) linear volatile polydialkylsiloxanes containing 2 to 9 silicon atoms and having a viscosity of less than or equal to 5×10⁻⁶ m²/s at 25°C. An example is decamethyltetrasiloxane sold in particular under the name SH 200 by the company Toray Silicone. Silicones belonging to this category are also described in the article published in Cosmetics and Toiletries, Vol. 91, Jan. 76, pp. 27-32, Todd & Byers, Volatile Silicone Fluids for Cosmetics*.* The viscosity of the silicones is measured at 25°C according to ASTM standard 445 Appendix C.

Non-volatile polydialkylsiloxanes may also be used. These non-volatile silicones are more particularly chosen from polydialkylsiloxanes, among which mention may be made mainly of polydimethylsiloxanes containing trimethylsilyl end groups.

Among these polydialkylsiloxanes, mention may be made, in a non-limiting manner, of the following commercial products::
- the Silbione^{®} oils of the 47 and 70 047 series or the Mirasil^{®} oils sold by Rhodia, for instance the oil 70 047 V 500 000;
- the oils of the Mirasil^{®} series sold by the company Rhodia;
- the oils of the 200 series from the company Dow Corning, such as DC200 with a viscosity of 60 000 mm²/s; and
- the Viscasil^{®} oils from General Electric and certain oils of the SF series (SF 96, SF 18) from General Electric.

Mention may also be made of polydimethylsiloxanes containing dimethylsilanol end groups known under the name dimethiconol (CTFA), such as the oils of the 48 series from the company Rhodia.

Among the silicones containing aryl groups, mention may be made of polydiarylsiloxanes, especially polydiphenylsiloxanes and polyalkylarylsiloxanes such as phenyl silicone oil.

The phenyl silicone oil may be chosen from the phenyl silicones of the following formula: 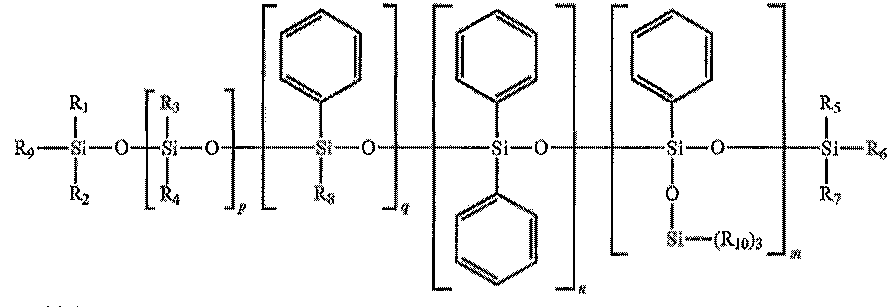 in which
R₁ to R₁₀, independently of each other, are saturated or unsaturated, linear, cyclic or branched C₁-C₃₀ hydrocarbon-based radicals, preferably C₁-C₁₂ hydrocarbon-based radicals, and more preferably C₁-C₆ hydrocarbon-based radicals, in particular methyl, ethyl, propyl, or butyl radicals, and
m, n, p, and q are, independently of each other, integers of 0 to 900 inclusive, preferably 0 to 500 inclusive, and more preferably 0 to 100 inclusive,
with the proviso that the sum n+m+q is other than 0.

Examples that may be mentioned include the products sold under the following names:
- the Silbione^{®} oils of the 70 641 series from Rhodia;
- the oils of the Rhodorsil^{®} 70 633 and 763 series from Rhodia;
- the oil Dow Corning 556 Cosmetic Grade Fluid from Dow Corning;
- the silicones of the PK series from Bayer, such as the product PK20;
- certain oils of the SF series from General Electric, such as SF 1023, SF 1154, SF 1250, and SF 1265.

As the phenyl silicone oil, phenyl trimethicone (R₁ to R₁₀ are methyl; p, q, and n = 0; m=1 in the above formula) is preferable.

The organomodified liquid silicones may especially contain polyethyleneoxy and/or polypropyleneoxy groups. Mention may thus be made of the silicone KF-6017 proposed by Shin-Etsu, and the oils Silwet^{®} L722 and L77 from the company Union Carbide.

The hydrocarbon oils may be chosen from:
- linear or branched, optionally cyclic, C₆-C₁₆ lower alkanes. Examples that may be mentioned include hexane, undecane, dodecane, tridecane, and isoparaffins, for instance isohexadecane, isododecane, and isodecane;
- linear or branched hydrocarbons containing more than 16 carbon atoms, such as liquid paraffins, liquid petroleum jelly, polydecenes and hydrogenated polyisobutenes such as Parleam^{®}, and squalane; and
- mixtures of alkanes, for example, C9-12 Alkane, C10-13 Alkane, C13-14 Alkane, C13-15 Alkane, C14-17 Alkane, C14-19 Alkane, C15-19 Alkane, C15-23 Alkane, C18-21Alkane, C8-9 Alkane/Cycloalkane, C9-10 Alkane/Cycloalkane, C9-11 Alkane/Cycloalkane, C9-16 Alkane/Cycloalkane, C10-12 Alkane/Cycloalkane, C11-14 Alkane/Cycloalkane, C11-15 Alkane/Cycloalkane, C12-13 Alkane/Cycloalkane.

As preferable examples of hydrocarbon oils, mention may be made of, for example, linear or branched hydrocarbons such as isohexadecane, isododecane, squalane, mineral oil (e.g., liquid paraffin), paraffin, vaseline or petrolatum, naphthalenes, and the like; hydrogenated polyisobutene, isoeicosan, and decene/butene copolymer; and mixtures thereof.

The term "fatty" in the fatty alcohol means the inclusion of a relatively large number of carbon atoms. Thus, alcohols which have 8 or more, preferably 10 or more, and more preferably 12 or more carbon atoms are encompassed within the scope of fatty alcohols. The fatty alcohol may be saturated or unsaturated. The fatty alcohol may be linear or branched.

The fatty alcohol may have the structure R-OH wherein R is chosen from saturated and unsaturated, linear and branched radicals containing from 8 to 40 carbon atoms, preferably from 10 to 30 carbon atoms, and more preferably from 12 to 20 carbon atoms. In at least one embodiment, R may be chosen from C₁₂-C₂₀ alkyl and C₁₂-C₂₀ alkenyl groups. R may or may not be substituted with at least one hydroxyl group.

As examples of the fatty alcohol, mention may be made of lauryl alcohol, cetyl alcohol, stearyl alcohol, isostearyl alcohol, behenyl alcohol, undecylenyl alcohol, myristyl alcohol, octyldodecanol, hexyldecanol, oleyl alcohol, linoleyl alcohol, palmitoleyl alcohol, arachidonyl alcohol, erucyl alcohol, and mixtures thereof.

It is preferable that the fatty alcohol be a saturated fatty alcohol.

Thus, the fatty alcohol may be selected from straight or branched, saturated or unsaturated C₈-C₃₀ alcohols, preferably straight or branched, saturated C₈-C₃₀ alcohols, and more preferably straight or branched, saturated C₁₂-C₂₀ alcohols.

The term "saturated fatty alcohol" here means an alcohol having a long aliphatic saturated carbon chain. It is preferable that the saturated fatty alcohol be selected from any linear or branched, saturated C₈-C₃₀ fatty alcohols. Among the linear or branched, saturated C₈-C₃₀ fatty alcohols, linear or branched, saturated C₁₂-C₂₀ fatty alcohols may preferably be used. Any linear or branched, saturated C₁₆-C₂₀ fatty alcohols may be more preferably used. Branched C₁₆-C₂₀ fatty alcohols may be even more preferably used.

As examples of saturated fatty alcohols, mention may be made of lauryl alcohol, cetyl alcohol, stearyl alcohol, isostearyl alcohol, behenyl alcohol, undecylenyl alcohol, myristyl alcohol, octyldodecanol, hexyldecanol, and mixtures thereof. In one embodiment, cetyl alcohol, stearyl alcohol, octyldodecanol, hexyldecanol, or a mixture thereof (e.g., cetearyl alcohol) as well as behenyl alcohol, can be used as a saturated fatty alcohol.

According to at least one embodiment, the fatty alcohol used in the composition according to the present invention is preferably chosen from cetyl alcohol, octyldodecanol, hexyldecanol, isostearyl alcohol, and mixtures thereof.

It is also preferable that the oil be chosen from oils with a molecular weight below 600 g/mol.

Preferably, the oil has a low molecular weight such as below 600 g/mol, chosen among ester oils with a short hydrocarbon chain or chains (C₁-C₁₂) (e.g., isopropyl myristate, isopropyl palmitate, isononyl isononanoate, and ethyl hexyl palmitate), silicone oils (e.g., volatile silicones such as cyclohexasiloxane), hydrocarbon oils (e.g., isododecane, isohexadecane, and squalane), branched and/or unsaturated fatty alcohol (C₁₂-C₃₀) type oils such as octyldodecanol and oleyl alcohol, and ether oils such as dicaprylyl ether.

It is preferable that the oil be chosen from polar oils, and more preferably from ester oils other than alkyl or alkylene carbonate, fatty alcohols, and a combination thereof. It is further preferred that the oil comprise both of ester oils and fatty alcohols, in particular the monoesters of monoacids and of monoalcohols represented by the formula R₁COOR₂ in which R₁ represents the residue of a linear fatty acid comprising from 10 to 20 carbon atoms, and R² represents a branched hydrocarbon-based chain containing from 2 to 8 carbon atoms and the fatty alcohol having the structure R-OH wherein R is chosen from saturated branched radicals containing from 4 to 40 carbon atoms, preferably from 6 to 30 carbon atoms, and more preferably from 12 to 20 carbon atoms.

The amount of the oil(s) in the composition according to the present invention may be 1% by weight or more, preferably 5% by weight or more, and more preferably 10% by weight or more; and may be 40% by weight or less, preferably 35% by weight or less, and more preferably 30% by weight or less, relative to the total weight of the composition.

The amount of the oil(s) in the composition according to the present invention may be from 1% to 40% by weight, preferably from 5% to 35% by weight, and more preferably from 10% to 30% by weight, relative to the total weight of the composition.

### • Water

The composition according to the present invention preferably includes water in the aqueous phase.

The amount of water in the composition according to the present invention may be 5% by weight or more, preferably 10% by weight or more, and more preferably 15% by weight or more; and may be 50% or less, preferably 40% by weight or less, and more preferably 30% by weight or less, relative to the total weight of the composition.

The amount of water in the composition according to the present invention may be from 5% to 50% by weight, preferably from 10% to 40% by weight, and more preferably from 15% to 30% by weight, relative to the total weight of the composition.

### • Monoalcohol

The composition according to the present invention may comprise at least one monoalcohol in the aqueous phase. Two or more types of monoalcohols may be used in combination.

Thus, a single type of monoalcohol or a combination of different types of monoalcohols may be used.

The monoalcohol forms the aqueous phase of the present invention. Therefore, the monoalcohol here can mean a water-soluble, hydrophilic monoalcohol. For the purpose of the present invention, the term "hydrophilic" here means that a substance is soluble in water at a concentration of at least 1% by weight relative to the total weight of the water at room temperature (25°C) and atmospheric pressure (10⁵ Pa).

The monoalcohol may be linear or branched, saturated or unsaturated mono-alcohols having from 1 to 8 carbon atoms, preferably from 2 to 8 carbon atoms, bearing only one hydroxyl (OH) function.

In one embodiment, the monoalcohol may be an aliphatic monoalcohol having from 1 to 8 carbon atoms, preferably from 2 to 8 carbon atoms.

The term "aliphatic monoalcohol" here means any linear or branched, saturated alkane compound bearing only one hydroxyl (OH) function.

The aliphatic monoalcohol(s) present in the compositions of the invention may be chosen from ethanol, propanol, butanol, isopropanol, isobutanol and mixtures thereof.

In one preferred embodiment of the present invention, the monoalcohol can be selected from linear aliphatic monoalcohol having from 1 to 8 carbon atoms, preferably from 2 to 8 carbon atoms, such as ethanol, propanol, butanol, and mixtures thereof.

The amount of the monoalcohol in the composition according to the present invention may be 1% by weight or more, preferably 3% by weight or more, and more preferably 5% by weight or more; and/or may be 20% or less, preferably 15% by weight or less, and more preferably 10% by weight or less, relative to the total weight of the composition.

The amount of monoalcohol in the composition according to the present invention may be from 1% to 20% by weight, preferably from 3% to 15% by weight, and more preferably from 5% to 10% by weight, relative to the total weight of the composition.

### • Lipophilic Thickener

The composition according to the present invention may comprise at least one lipophilic thickener. Two or more lipophilic thickeners may be used in combination. Thus, a single type of the lipophilic thickener or a combination of different types of the lipophilic thickeners may be used.

For the purposes of the present invention, the term "lipophilic" here can mean substances having a solubility of at least 1 g/L, preferably at least 10 g/L, and more preferably at least 100 g/L, in an oil at room temperature (25°C) and atmospheric pressure (105 Pa). In another aspect, the term "lipophilic" can refer to substances which are not soluble in water or which have a solubility of 1 g/L or less, or 0.1 g/L or less in water, at 25 °C and atmospheric pressure.

The term "lipophilic thickener" means an agent, inorganic or organic, in a particulate form or not, able to gel the oils of the composition. The term "particulate lipophilic thickener" means a lipophilic thickener in the form of particles or of crystals (particulate or crystalline).

The lipophilic thickener(s) used in the composition according to the invention may be chosen from mineral lipophilic thickeners and organic lipophilic thickeners, and mixtures of these compounds. The lipophilic thickener is preferably particulate.

The mineral lipophilic thickeners that may be used in the composition according to the invention are preferably mineral particles constituted essentially of mineral oxides and/or hydroxides.

These particles are preferably insoluble in water at room temperature (25°C). The term "insoluble" means a solubility of less than 0.5% by weight.

Preferably, the number-average primary size of these mineral particles ranges from 0.01 to 500 µm, it preferably ranges from 0.1 to 200 µm, and even more preferentially it ranges from 1 to 100 µm.

For the purposes of the present invention, the term "primary particle size" means the maximum dimension that it is possible to measure between two diametrically opposite points on an individual particle.

The size of the mineral particles may be determined by transmission electron microscopy or by measuring the specific surface area via the BET method or by laser particle size analysis.

The mineral particles that may be used in accordance with the invention may be in various forms, for example in the form of spheres, needles, flakes or platelets.

In a preferred variant of the invention, the mineral lipophilic thickener(s) are platelet-shaped particles.

The mineral lipophilic thickener(s) that may be used in the cosmetic composition according to the invention may preferably be chosen from silicas and silicates.

The silicates of the invention may be natural or chemically modified (or synthetic).

Silicates correspond to optionally hydrated silica in which some of the silicon atoms are replaced with metal cations such as Al³⁺, B³⁺, Fe³⁺, Ga³⁺, Be²⁺, Zn²⁺, Mg²⁺, Co³⁺, Ni³⁺, Na⁺, Li⁺, Ca²⁺, Cu²⁺.

More particularly, the silicates that may be used in the context of the invention are chosen from clays of the smectite family such as montmorillonites, hectorites, bentonites, beidellites and saponites, and also of the vermiculite, stevensite and chlorite families.

These clays may be of natural or synthetic origin. Clays that are cosmetically compatible and acceptable with keratin materials are preferably used.

The silicate may be chosen from montmorillonite, bentonite, hectorite, attapulgite and sepiolite, and mixtures thereof. The silicate(s) are preferably chosen from bentonites and hectorites.

The silicates may be modified with a compound chosen from quaternary amines, tertiary amines, amine acetates, imidazolines, amine soaps, fatty sulfates, alkylarylsulfonates and amine oxides, and mixtures thereof.

As silicates that are suitable for use, mention may be made of quaternium-18 bentonites, such as those sold under the names Bentone 3, Bentone 38 and Bentone 38V by Rheox, Tixogel VP by United Catalyst and Claytone 34, Claytone 40 and Claytone XL by Southern Clay; stearalkonium bentonites, such as those sold under the names Bentone 27 by Rheox, Tixogel LG by United Catalyst and Claytone AF and Claytone APA by Southern Clay; quaternium-18/benzalkonium bentonites, such as those sold under the names Claytone HT and Claytone PS by Southern Clay; quaternium-18 hectorites, such as those sold under the names Bentone Gel DOA, Bentone Gel ECO5, Bentone Gel EUG, Bentone Gel IPP, Bentone Gel ISD, Bentone Gel SS71, Bentone Gel VS8 and Bentone Gel VS38 by Rheox, and Simagel M and Simagel SI 345 by Biophil.

The silicates that may be used in the composition according to the invention may be chosen, in particular, from modified hectorites such as hectorite modified with a C₁₀-C₁₂ fatty acid ammonium chloride, especially distearyldimethylammonium chloride and stearylbenzyldimethylammonium chloride.

As explained previously, the mineral lipophilic thickener(s) that may be used in the composition according to the invention may be silicas.

The silicas that may be used in the composition according to the invention are fumed silicas.

The fumed silicas may be obtained by high-temperature hydrolysis of a volatile silicon compound in an oxyhydrogen flame, producing a finely divided silica. This process makes it possible in particular to obtain hydrophilic silicas bearing a large number of silanol groups at their surface. Such hydrophilic silicas are sold, for example, under the names Aerosil 130^{®}, Aerosil 2000, Aerosil 255^{®}, Aerosil 300^{®} and Aerosil 380^{®} by the company Degussa, and Cab-O-Sil HS-5^{®}, Cab-O-Sil EH-5^{®}, Cab-O-Sil LM-130^{®}, Cab-O-Sil MS-55^{®} and Cab-O-Sil M-5^{®} by the company Cabot.

It is possible to chemically modify the surface of said silicas, via a chemical reaction generating a reduction in the number of silanol groups. It is especially possible to substitute silanol groups with hydrophobic groups: a hydrophobic silica is then obtained.

The hydrophobic groups may be:
(a) trimethylsiloxy groups, which are obtained especially by treating fumed silica in the presence of hexamethyldisilazane. Silicas thus treated are known as Silica silylate according to the CTFA (6th Edition, 1995). They are sold, for example, under the references Aerosil R812^{®} by the company Degussa, and Cab-O-Sil TS-530^{®} by the company Cabot;
(b) dimethylsilyloxy or polydimethylsiloxane groups, which are obtained in particular by treating fumed silica in the presence of polydimethylsiloxane or dimethyldichlorosilane.

Silicas thus treated are known as silica dimethyl silylate according to the CTFA (6th Edition, 1995). They are sold, for example, under the references Aerosil R972^{®} and Aerosil R974^{®} by the company Degussa, and Cab-O-Sil TS-610^{®} and Cab-O-Sil TS-720^{®} by the company Cabot.

Preferentially, the mineral lipophilic thickeners are chosen from hectorites modified with a C₁₀-C₁₂ fatty acid ammonium chloride, especially distearyldimethylammonium chloride and stearylbenzyldimethylammonium chloride, and hydrophilic fumed silicas such as the hydrophilic silicas sold under the name Aerosil 200^{®}.

More preferentially, the mineral lipophilic thickeners are chosen from hectorites modified with a C₁₀-C₁₂ fatty acid ammonium chloride, especially hectorite modified with distearyldimethylammonium chloride (or disteardimonium hectorite), such as the product sold under the name Bentone 38VCG by Elementis, and the hectorite modified with stearylbenzyldimethylammonium chloride, such as the product sold under the name Bentone 27V by Elementis.

As explained previously, the lipophilic thickener(s) that may be used in the composition according to the invention may also be chosen from organic lipophilic thickeners.

Preferably, the organic lipophilic thickener(s) are chosen from semi-crystalline polymers, non-silicone polyamides, silicone polyamides, saccharide or polysaccharide monoalkyl or polyalkyl esters, N-acylamino acid amide derivatives, polymers comprising an alkylene and/or styrene block, such as polystearyl acrylates, elastomeric organopolysiloxanes, solid fatty esters, in particular C₈-C₃₀ and preferably C₁₈-C₂₄ fatty acid esters, and mixtures of these compounds. These copolymers may be diblock, triblock or multi-block polymers, radial-block polymers, also known as star copolymers, or alternatively comb polymers.

Among the C₈-C₃₀ and preferably C₁₈-C₂₄ fatty acid esters, mention may be made of mono-, di- or triesters of C₈-C₃₀ and preferably C₁₈-C₂₄ fatty acids and of polyols, more particularly mono-, di- or triesters of C₈-C₃₀ and preferably C₁₈-C₂₄ fatty acids and of glycerol. Use may especially be made of a mixture of these compounds such as a mixture of mono-, di- and triesters of behenic acid and of glycerol.

Most particularly, the organic lipophilic thickener(s) are chosen from semi-crystalline polymers, non-silicone polyamides, silicone polyamides, polymers comprising an alkylene and/or styrene block, such as polystearyl acrylates, solid fatty esters, in particular C₈-C₃₀ and preferably C₁₈-C₂₄ fatty acid esters, and mixtures of these compounds.

Even more preferentially, the organic lipophilic thickener(s) are chosen from C₈-C₃₀ and preferably C₁₈-C₂₄ fatty acid esters and mixtures thereof, better still esters of C₈-C₃₀ and preferably C₁₈-C₂₄ fatty acids and of polyols, more particularly mono-, di- or triesters of C₈-C₃₀ and preferably C₁₈-C₂₄ fatty acids and of glycerol.

The lipophilic thickener(s) may be present in an amount of 0.1% by weight or more, preferably 0.2% by weight or more, more preferably 0.5% by weight or more, and/or may be present in an amount of 5% by weight or less, preferably 3% by weight or less, and more preferably 2% by weight or less, relative to the total weight of the composition.

The amount of the lipophilic thickener(s) in the composition according to the present invention may be from 0.1% to 5% by weight, preferably from 0.2% to 3% by weight, and more preferably from 0.5% to 2% by weight, relative to the total weight of the composition.

### • Surfactant

The composition according to the present invention may comprise at least one surfactant chosen from amphoteric, anionic, cationic, or nonionic surfactants other than polyoxyalkylenated silicone, used alone or as a mixture.

Examples of anionic surfactants usable in the compositions of the invention may include alkyl sulfates, alkyl ether sulfates, alkyl sulfonates, alkyl ether sulfonates, sulfate esters of an alkylphenoxy polyoxyethylene ethanol, alpha-olefin sulfonates, beta alkyloxy alkene sulfonates, alkyl arylsulfonates, alkyl carbonates, succinamates, sulfosuccinates, sarcosinates, octoxynol or nonoxynol phosphates, taurates, fatty taurides, sulfated monoglycerides, fatty acid amino polyoxyethylene sulfates, isethionates, alkyl benzene sulfonic acids, polyoxyethylene alkyl ether sulfuric acids, polyoxyethylene alkyl ether carboxylic acids, and polyoxyethylene alkyl amide ether carboxylic acids, and salts thereof.

Examples of the nonionic surfactants usable in the compositions of the invention may include polyethoxylated fatty alcohols or polyglycerolated fatty alcohols, such as the adducts of ethylene oxide with lauryl alcohol, especially those containing from 9 to 50 oxyethylene units (Laureth-9 to Laureth-50 as the INCI names), in particular Laureth-9; esters of polyols and of a fatty acid possessing a saturated or unsaturated chain comprising, for example, from 8 to 24 carbon atoms, and their oxyalkylenated derivatives, that is to say comprising oxyethylene and/or oxypropylene units, such as esters of glycerol and of a C₈-C₂₄ fatty acid, and their oxyalkylenated derivatives, in particular polyoxyethylenated glyceryl stearate (mono-, di- and/or tristearate), for examples PEG-30 dipolyhydroxystearate and PEG-20 glyceryl triisostearate; esters of sugar and of a C₈-C₂₄ fatty acid and their oxyalkylenated derivatives, such as sorbitol esters of C₈-C₂₄ fatty acids and polyoxyalkylenated derivatives; ethers of a sugar and of C₈-C₂₄ fatty alcohols, such as caprylyl/capryl glucoside; polyoxyethylene alkyl ethers; polyoxyethylene oxypropylene alkyl ethers; fatty acid alkanol amides; alkyl amine oxides; alkyl polyglycosides and silicone surfactants other than polyoxyalkylenated silicone.

The sorbitol esters of C₈-C₂₄ fatty acids and polyoxyalkylenated derivatives thereof can be selected from sorbitan palmitate, sorbitan isostearate, sorbitan trioleate and esters of fatty acids and alkoxylated sorbitan containing for example from 20 to 100 EO, such as for example sorbitan monostearate (CTFA name: sorbitan stearate), sold by the company ICI under the name Span 60, sorbitan monopalmitate (CTFA name: sorbitan palmitate), sold by the company ICI under the name Span 40, and sorbitan tristearate 20 EO (CTFA name: polysorbate 65), sold by the company ICI under the name Tween 65, polyethylene sorbitan trioleate (polysorbate 85) or the compounds marketed under the trade names Tween 20 or Tween 60 by Uniqema.

Examples of amphoteric surfactants usable in the compositions of the invention may include alkanoyl amide propyl-N,N-dimethyl glycine betaines, alkanoyl amide propyl-N,N-dimethyl-2-hydroxypropyl sulfobetaines, alkyl-N,N-dimethyl glycine betaines, alkanoyl amide propyl-N,N-dimethyl-propyl sulfobetaines, lauryl-N,N-dimethyl-2-hydroxypropyl sulfobetaines, and salts thereof.

Examples of cationic surfactants usable in the compositions of the invention may include C₈-C₂₄ long-chain di-alkyl dimethyl ammonium salts, long-chain mono-alkyl monobenzyl dimethyl ammonium salts and long-chain mono-alkyl trimethyl ammonium salts, all of which may have amide or ester linkages therein, and the counter ions are preferably halogen atoms such as chlorine and bromine atoms, sulfates, and alkyl group-containing sulfate residues such as methyl- and ethyl- sulfuric acid, and salts thereof. Cationic surfactants of an amine type include long-chain di-alkyl monomethyl amine salts with a long-chain C₈-C₂₄ alkyl group which may have an amide or ester linkage therein, preferably in the form of hydrochlorides, sulfates or phosphates, and salts thereof.

The surfactant(s) may be present in the composition in a content of 0.5% by weight or more, preferably 1% by weight or more, and more preferably 2% by weight or more; and/or may be present in the composition in a content of 15% by weight or less, preferably from 10% by weight or less, and more preferably 7% by weight or less, relative to the total weight of the composition.

The amount of the surfactant(s) in the composition according to the present invention may be from 0.5% to 15% by weight, preferably from 1% to 10% by weight, and more preferably from 3% to 7% by weight, relative to the total weight of the composition.

### • UV filter

The composition according to the present invention may comprise at least one UV filter.

The UV filter may be solid or liquid, preferably liquid. The terms "solid" and "liquid" mean solid and liquid, respectively, at 25°C under 1 atm. The UV filter may be made from at least one organic or inorganic material, preferably at least one organic material. Thus, the UV filter is preferably an organic UV filter, which may be included in the oily phase.

The organic UV filter may be selected from the group consisting of anthranilic derivatives; dibenzoylmethane derivatives; cinnamic derivatives; salicylic derivatives such as homosalate (homomenthyl salicylate) and ethylhexyl salicylate; camphor derivatives; benzophenone derivatives; β,β-diphenylacrylate derivatives; triazine derivatives; benzotriazole derivatives; benzalmalonate derivatives; benzimidazole derivatives; imidazoline derivatives; bis-benzoazolyl derivatives; p-aminobenzoic acid (PABA) and derivatives thereof; benzoxazole derivatives; screening polymers and screening silicones; dimers derived from α-alkylstyrene; 4,4-diarylbutadienes; octocrylene and derivatives thereof, guaiazulene and derivatives thereof, rutin and derivatives thereof, flavonoids, biflavonoids, oryzanol and derivatives thereof, quinic acid and derivatives thereof, phenols, retinol, cysteine, aromatic amino acids, peptides having an aromatic amino acid residue, and mixtures thereof.

The UV filter(s) may be present in the composition in a content of 0.5% by weight or more, preferably 1% by weight or more, and more preferably 3% by weight or more, and/or it may be present in the composition in a content of 15% by weight or less, preferably 10% by weight or less, and more preferably 7% by weight or less, relative to the total weight of the composition.

The amount of the UV filter(s) in the composition according to the present invention may be from 0.5% to 15% by weight, preferably from 1% to 10% by weight, and more preferably from 3% to 7% by weight, relative to the total weight of the composition.

### • Filler

The composition according to the present invention may comprise at least one filler other than the (a) spherical hydrophobic silica aerogel. Two or more fillers can be combined. The filler can be inorganic or organic, preferably inorganic.

As the inorganic filler mention may be made of talc, mica, silica, hollow silica, magnesium aluminum silicate, titanium dioxide, kaolin, bentone, calcium carbonate, magnesium hydrogen carbonate, hydroxyapatite, boron nitride, fluorphlogopite, sericite, calcinated talc, calcinated mica, calcinated sericite, synthetic mica, perlite, lauroyl lysine, metal soap, bismuth oxychloride, barium sulfate, magnesium sulfate, magnesium carbonate, and mixtures thereof, optionally hydrophilic- or hydrophobic-treated.

The inorganic filler may be composite silica particle. In the context of the present invention, the term "composite silica particles" means silica particles within which functional compounds, preferably metal oxides, are included. Therefore, preferably, the composite silica particles may refer to "metal oxide-including silica particles". Most preferably, the metal oxides are scattered inside the silica particles.

The metal oxides may preferably be chosen from titanium oxide, zinc oxide, iron oxide and zirconium oxide, or mixtures thereof, and more particularly from titanium dioxide (TiO₂) and zinc oxide, and mixtures thereof. Particularly preferably, titanium dioxide may be used. In this embodiment, the composite silica particle is referred to Silica (and) Titanium Dioxide.

The composite silica particles may have an average particle size as determined by an image analysis method of 0.1 µm or more, preferably 0.5 µm or more, and more preferably 1 µm or more, and may have an average particle size by an image analysis method of 50 µm or less, preferably 20 µm or less, and more preferably 12 µm or less.

The "average particle size" can be determined in accordance with the following procedure: Particle sizes of 50 particles are measured using a SEM image and an average value of the particle sizes is calculated.

The composite silica particles may be porous or non-porous, and they may have a low oil-absorbing capacity.

In the composite silica particles, the weight ratio of silica to the functional compounds (preferably metal oxides and most preferably titanium dioxide) may be from 9:1 to 5:5, preferably from 4:1 to 3:2, and more preferably 7:3.

The composite silica particles may be surface-treated to be hydrophobic. For example, the composite silica particle may be surface-treated with alkylsilanes.

According to the present invention, the inorganic filler may have been surface-treated with least one silicone oil and/or at least one non-silicone oil.

In one embodiment of the present invention, the inorganic filler can be colorants or pigments. The term "pigments" should be understood to mean white or colored, mineral or organic particles of any shape, which are insoluble in a physiological medium, and which are intended to color the composition. The pigments may be white or colored, and mineral and/or organic. The mean particle size of the coated pigment is in general 100 nm or more.

Among the mineral pigments that may be mentioned are titanium dioxide, such as pigmentary titanium dioxide rutile type, optionally surface-treated, zirconium oxide or cerium oxide, and also zinc oxide, iron (black, yellow or red) oxide or chromium oxide, silica, mica, fluorphlogopite, sericite, kaolin, aluminum hydroxide, manganese violet, ultramarine blue, chromium hydrate and ferric blue, and metal powders, for instance aluminum powder and copper powder.

The pigments can be composite pigments. The composite pigments may comprise at least one metal oxide and at least one aluminum oxide (or alumina). According to one particular form of the present invention, the composite pigment may also additionally comprise silica particles. According to one particular form of the present invention, the composite pigment may also additionally comprise titanium dioxide. Among the composite pigments that may be mentioned are Covalumine Sonoma Red AS^{®} (INCI name: Alumina (and) CI 77491 (and) Triethoxycaprylylsilane); Covalumine Sonoma Yellow AS^{®} (INCI name: Alumina (and) CI 77492 (and) Triethoxycaprylylsilane); Covalumine Sonoma Black AS^{®} (INCI name: Alumina (and) CI 77499 (and) Triethoxycaprylylsilane) (and) silica); and Covalumine Atlas White AS^{®} (INCI name: Alumina (and) Titanium Oxide (and) Triethoxycaprylylsilane), and that of INCI name: Titanium Dioxide (and) Triethoxycaprylylsilane (and) Alumina (and) Silica.

The pigments may be surface treated, for example. Surface treatment compounds may include a hydrophobic agent, such as silanes, preferably a C₁-C₂₀-alkylsilane, more preferentially a tri(C₁-C₄)alkoxy(C₁-C₁₂)alkylsilane such as triethoxycaprylylsilane, silicones, such as organosilicone, di-organosilicone, dimethicones, hydrogen dimethicone, methicones, polyurethanes, silicone-polyurethanes, and fluoro- or perfluoro-derivatives thereof, fatty acid soaps, C9-15 fluoroalcohol phosphates, acrylate/dimethicone copolymers, mixed C9-C15 fluoroalcohol phosphate/silicone copolymers, lecithins or hydrogenated lecithin, waxes, such as carnauba wax, polyethylene, chitosan and optionally acylated amino acids, such as lauroyl lysine, disodium stearoyl glutamate and aluminum acyl glutamate. Other hydrophobic agents may include isopropyl titanium triisostearate (ITT), ITT and dimethicone (ITT/dimethicone) cross-polymers, ITT and amino acid, ITT/triethoxycaprylylsilane crosspolymer, fatty acids (e.g., stearates), HDI/trimethylol hexyllactone crosspolymer, PEG-8 methyl ether triethoxysilane, aloe, jojoba ester, and Magnesium Myristate (MM).

As the organic filler, mention may be made of acrylic polymer powders, silicone powders, wax powders, polyamide powders, urethane polymer powders, tetrafluoroethylene polymer powders, polyacrylonitrile powders, poly-β-alanine powders, polyethylene powders, polytetrafluoroethylene powders, (meth)acrylic or (meth)acrylate powders, lauroyllysine, starch, cellulose powder, tetrafluoroethylene polymer powders and mixtures thereof.

The (meth)acrylic or (meth)acrylate powders can include, for example, polymethylmethacrylate crosspolymer, methyl methacrylate/glycol dimethacrylate crosspolymer, polymethyl methacrylate/ethylene glycol dimethacrylate powders, polyallyl methacrylate/ethylene glycol dimethacrylate powders, and ethylene glycol dimethacrylate/lauryl methacrylate copolymer powders.

As the polyamide powders, mention may be made of those sold under the name "Orgasol" by the company Atochem. These polyamide powder particles are moreover known according to their various physicochemical properties under the name "Nylon 12" or "Nylon 6". The polyamide powders useful in the present invention may also include those sold under the name SP500 by the company TORAY.

The filler(s) may be present in the composition in a content of 1% by weight or more, preferably 5% by weight or more, and more preferably 10% by weight or more, and/or it may be present in the composition in a content of 30% by weight or less, preferably 25% by weight or less, and more preferably 20% by weight or less, relative to the total weight of the composition.

The amount of the filler(s) in the composition according to the present invention may be from 1% to 30% by weight, preferably from 5% to 25% by weight, and more preferably from 10% to 20% by weight, relative to the total weight of the composition.

### • Skincare Active Agent

The composition according to the present invention may comprise at least one skincare active agent. If two or more skincare active agents are used, they may be the same or different.

It is preferable that the skin care active ingredient be a skin care cosmetic active ingredient, and more preferably a skin peeling agent, a skin whitening agent, or a skin anti-aging agent such as an anti-wrinkle agent.

As the skin care active ingredient, mention may be made of Vitamin B3 and derivatives, ascorbic acid and derivatives thereof, resorcinol derivatives, C-glycoside derivatives, salicylic acid and derivatives thereof, α-hydroxy acids, niacinamide and mixtures thereof.

The skin care active ingredient(s) may be present in the composition in a content of 0.3% by weight or more, preferably 1% by weight or more, and more preferably 3% by weight or more, and/or it may be present in the composition in a content of 15% by weight or less, preferably 10% by weight or less, and more preferably 7% by weight or less, relative to the total weight of the composition.

The amount of the skin care active ingredient(s) in the composition according to the present invention may be from 0.3% to 15% by weight, preferably from 1% to 10% by weight, and more preferably from 3% to 7% by weight, relative to the total weight of the composition.

### •Additives

The composition according to the present invention may also comprise any other optional additive(s) usually used in the field of cosmetics, chosen, for example, from cationic, nonionic or amphoteric polymers, hydrophobic organic solvents, gums, dyes, resins, thickeners, film-forming agents other than the spherical hydrophobic silica aerogel, dispersants, antioxidants, preserving agents such as phenoxyethanol, fragrances, neutralizers, pH adjusting agents, antiseptics, other cosmetic active agents, vitamins such as tocopherol, moisturizers, chelating agents, emollients or collagen-protecting agents, and mixtures thereof.

The composition according to the present invention preferably has a viscosity of less than 5000 mPa·s, more preferably less than 3,000 mPa·s; and/or preferably has a viscosity more than 300 mPa·s, and more preferably more than 500 mPa·s at room temperature (25°C).

For the viscosity measurement, VISCOMAN^{™} (GILSON Technology) can be used. The bulk viscosity (mPaS, PaS) is automatically calculated from the needed aspiration force in a small pipet (10 µL).

The composition according to the present invention can be prepared by mixing the above-described essential and optional ingredients in a conventional manner. In the case that at least one of the above ingredients is solid at room temperature, the ingredient can be heated until it is dissolved. It is possible to further comprise mixing any of the optional ingredients and heating the composition until the ingredient is dissolved.

### [Process and Use]

The composition according to the present invention is intended to be used as a cosmetic composition. Therefore, the cosmetic composition according to the present invention may be intended for application onto keratin substances, for example the skin, scalp, hair, mucosa such as lips, and nails, in particular the skin, for instance that of the face.

The composition according to the present invention may be used as a skin cosmetic composition, preferably a skin makeup composition, and more preferably a foundation.

Thus, the present invention also relates to a non-therapeutic cosmetic method or process, preferably for caring for and/or conditioning keratinous substance, comprising:
applying onto the keratinous substance, such as the skin, scalp, and lips, in particular facial skin a composition having at least one aqueous phase and at least one oily phase, comprising:
   (a) at least one spherical hydrophobic silica aerogel;
   (b) at least one thickening aid selected from polyoxyalkylenated silicone and alkyl or alkylene carbonate;
   (c) at least one film-forming polymer;
   (d) at least one anionic polymer; and
   (e) at least one polyol in an amount of 6% by weight or more relative to the total weight of the composition,
wherein the aqueous phase is present in an amount of 40% by weight or more relative to the total weight of the composition.

The present invention also relates to a use of (a) at least one spherical hydrophobic silica aerogel, (b) at least one thickening aid selected from polyoxyalkylenated silicone and alkyl or alkylene carbonate, (c) at least one film-forming polymer, and (e) at least one anionic polymer for stabilizing an emulsion having at least one aqueous phase and at least one oily phase, comprising (d) at least one polyol in an amount of 6% by weight or more relative to the total weight of the composition, wherein the aqueous phase is present in an amount of 40% by weight or more relative to the total weight of the composition.

The same explanations given for the composition, (a) at least one spherical hydrophobic silica aerogel, (b) at least one thickening aid selected from polyoxyalkylenated silicone and alkyl or alkylene carbonate, (c) at least one film-forming polymer, (d) at least one polyol, and (e) at least one anionic polymer can be applied to those for the method, process, and use according to the present invention. The composition used in the process and use according to the present invention may include any of the optional ingredients explained above for the composition according to the present invention.

### EXAMPLES

The present invention will be described in a more detailed manner by way of examples. However, these examples should not be construed as limiting the scope of the present invention.

### Preparation method of inventive and comparative W/O foundation compositions

The ingredients listed as A1 in Tables 1 and 2 were completely mixed at 45°C. Disteardimonium hectorite listed as A2 was then added and they were mixed with a Moritz homogenizer at 3,500 rpm for 5 minutes at 45°C. The ingredients as listed C were added and mixed at 3,500 rpm for 10 minutes at 45°C. A mixture of the ingredients was cooled down to 25°C. The ingredients listed as D was added and mixed at 3,000 rpm for 2 minutes at room temperature, and then the ingredients listed as E was added and dispersed at 2,000 rpm for 5 minutes at room temperature to obtain the emulsion compositions.

The spherical silica silylate aerogel was obtained from Tokuyama and had 10 µm of the average primary particle size, 0.88 of an average circularity, 592 m²/g of BET specific surface area, 4.0 ml/g of a pore volume determined by BJH method, 6.8 mL/g of an oil absorption capacity measured with JIS-K6217-4, and 20 nm of a peak pore radius determined by BJH method.

### Evaluation

### [Stability]

Each of the compositions was left to stand at 45°C for up to two months. The stability was evaluated from a change in appearance, in particular a separation between aqueous and oily phases. The criteria was as follows.

| | |
|---|---|
| Excellent: | No separation was observed for two months with no viscosity reduction. |
| Very good: | No separation was observed for two months with viscosity reduction. |
| Good: | No separation was observed for one month with no viscosity reduction. |
| Fair: | No separation was observed for one month with viscosity reduction. |
| Bad | Separation was observed for one month with viscosity reduction. |

### [Sensory Assessments]

10 professional panelists evaluated the sensory aspects of "water-splashing sensation", "moist sensation", and "moisture lastingness" in accordance with the following criteria. "Water-breaking sensation" was assessed during application of the composition on the face. "Moist sensation" was assessed 5 minutes later after the composition was applied on the face. "Moisture lastingness" was assessed 6 hours later after the composition was applied on the face.

### (Water-splashing sensation)

| | |
|---|---|
| Excellent: | 9-10 panels of 10 panels felt water-splashing sensation. |
| Very good: | 7-8 panels of 10 panels felt water-splashing sensation. |
| Good: | 5-6 panels of 10 panels felt water-splashing sensation. |
| Fair: | 3-4 panels of 10 panels felt water-splashing sensation. |
| Bad: | 0-2 panels of 10 panels felt water-splashing sensation. |

### (Moist sensation)

| | |
|---|---|
| Excellent: | 9-10 panels of 10 panels felt moist sensation. |
| Very good: | 7-8 panels of 10 panels felt moist sensation. |
| Good: | 5-6 panels of 10 panels felt moist sensation. |
| Fair: | 3-4 panels of 10 panels felt moist sensation. |
| Bad: | 0-2 panels of 10 panels felt moist sensation. |

### (Moisture lastingness)

| | |
|---|---|
| Excellent: | 9-10 panels of 10 panels felt moisture at 6 hours after application. |
| Very good: | 7-8 panels of 10 panels felt moisture at 6 hours after application. |
| Good: | 5-6 panels of 10 panels felt moisture at 6 hours after application. |
| Fair: | 3-4 panels of 10 panels felt moisture at 6 hours after application. |
| Bad: | 0-2 panels of 10 panels felt moisture at 6 hours after application. |

The results are shown in Tables 1 and 2.

**Table 1**

| Ingredient | | Ex.1 | Ex. 2 | Ex. 3 | Ex.4 | Ex.5 | Ex.6 | Ex.7 |
|---|---|---|---|---|---|---|---|---|
| A1 | PEG-30 dipolyhydroxystearate | 2.2 | 2.2 | 2.2 | 2.2 | 2.2 | 2.2 | 2.2 |
| | Sorbitan isostearate | 1.6 | 1.6 | 1.6 | 1.6 | 1.6 | 1.6 | 1.6 |
| | Ethylhexyl salicylate | 5 | 5 | 5 | 5 | 5 | 5 | 5 |
| | Homosalate | 5 | 5 | 5 | 5 | 5 | 5 | 5 |
| | Dimethicone | 9.2 | 9.2 | 9.2 | 9.2 | 9.2 | 9.2 | 9.2 |
| | Phenyl trimethicone | 2 | 2 | 2 | 2 | 2 | 2 | 2 |
| | Undecane (and) Tridecane | 6.6 | 6.6 | 6.6 | 6.6 | 6.6 | 6.6 | 6.6 |
| | Diisopropyl sebacate | 1.6 | 1.6 | 1.6 | 1.6 | 1.6 | 1.6 | 1.6 |
| | Tocopherol | 0.11 | 0.11 | 0.11 | 0.11 | 0.11 | 0.11 | 0.11 |
| | Trimethylsiloxysilicate | 6.7 | 6.7 | 6.7 | 6.7 | 6.7 | 6.7 | 6.7 |
| | PEG-10 Dimethicone | 0.4 | - | 0.4 | 0.4 | 0.4 | 0.4 | 0.4 |
| | Propylene carbonate | - | 0.4 | - | - | - | - | - |
| A2 | Disteardimonium hectorite | 1.2 | 1.2 | 1.2 | 1.2 | 1.2 | 1.2 | 1.2 |
| B | Perlite | 0.1 | 0.1 | 0.1 | 0.1 | 0.1 | 0.1 | 0.1 |
| | Silica silylate | 0.5 | 0.5 | 0.5 | 0.5 | 0.5 | 0.5 | 0.5 |
| | Silica (and) titanium dioxide | 0.5 | 0.5 | 0.5 | 0.5 | 0.5 | 0.5 | 0.5 |
| C | Glycerin | 5 | 5 | 5 | 3 | 5 | 5 | 5 |
| | Butylene glycol | 8 | 8 | 8 | 3 | 8 | 8 | 8 |
| | Phenoxyethanol | 0.5 | 0.5 | 0.5 | 0.5 | 0.5 | 0.5 | 0.5 |
| | Sodium hyaluronate (Mw: 35,000) | 1 | 1 | - | 1 | 3 | 0.5 | 0.1 |
| | Acetylated sodium hyaluronate | - | - | 1 | - | - | - | - |
| | Water | qs 100 | qs 100 | qs 100 | qs 100 | qs 100 | qs 100 | qs 100 |
| | Niacinamide | 5 | 5 | 5 | 5 | 5 | 5 | 5 |
| | Magnesium sulfate | 0.3 | 0.3 | 0.3 | 0.3 | 0.3 | 0.3 | 0.3 |
| D | Ethanol | 6.8 | 6.8 | 6.8 | 6.8 | 6.8 | 6.8 | 6.8 |
| E | Titanium dioxide (and) alumina (and) dimethicone (and) isopropyl titanium triisostearate | 12.6 | 12.6 | 12.6 | 12.6 | 12.6 | 12.6 | 12.6 |
| | Iron oxides (and) isopropyl titanium triisostearate | 1.94 | 1.94 | 1.94 | 1.94 | 1.94 | 1.94 | 1.94 |
| Water Phase Content (wt%) | | 43 | 43 | 43 | 43 | 43 | 43 | 43 |
| Stability | | Very good | Very good | Very good | Very good | Very good | Very good | Good |
| Water-splashing sensation | | Very good | Very good | Very good | Very good | Excell ent | Very good | Very good |
| Moist sensation | | Very good | Very good | Very good | Good | Very good | Very good | Very good |
| Moisture lastingness | | Very good | Very good | Very good | Good | Very good | Very good | Very good |

**Table 2**

| Ingredient | | Ex.8 | Ex. 9 | Comp . Ex. 1 | Comp . Ex. 2 | Comp . Ex. 3 | Comp Ex. 4 | Comp. Ex. 5 | Comp. Ex. 6 |
|---|---|---|---|---|---|---|---|---|---|
| A1 | PEG-30 dipolyhydroxystearate | 2.2 | 2.5 | 2.2 | 2.2 | 2.2 | 2.2 | 2.2 | 2.2 |
| | Sorbitan isostearate | 1.6 | 1.9 | 1.6 | 1.6 | 1.6 | 1.6 | 1.6 | 1.6 |
| | Ethylhexyl salicylate | 5 | 5.9 | 5 | 5 | 5 | 5 | 5 | 3.3 |
| | Octocrylene | - | - | - | - | - | - | - | 3.3 |
| | Homosalate | 5 | 5.9 | 5 | 5 | 5 | 5 | 5 | 4.4 |
| | Dimethicone | 9.2 | 10.8 | 9.2 | 15.9 | 9.7 | 15.7 | 9.2 | 10.1 |
| | Phenyl trimethicone | 2 | 2.3 | 2 | 2 | 2 | 2 | 2 | - |
| | Isododecane | - | - | - | - | - | - | - | 1.1 |
| | Undecane (and) Tridecane | 6.6 | 7.8 | 6.6 | 6.6 | 6.6 | 6.6 | 6.6 | 6.6 |
| | Diisopropyl sebacate | 1.6 | 1.9 | 1.6 | 1.6 | 1.6 | 1.6 | 1.6 | 1.6 |
| | Tocopherol | 0.11 | 0.13 | 0.1 | 0.1 | 0.1 | 0.1 | 0.1 | 0.1 |
| | Trimethylsiloxysilicate | 6.7 | 7.8 | 6.7 | - | 6.7 | 6.7 | 6.7 | 7.3 |
| | PEG-10 Dimethicone | 0.4 | 0.4 | - | 0.4 | 0.4 | 0.4 | 0.4 | - |
| A2 | Disteardimonium hectorite | 1.2 | 1.4 | 1.2 | 1.2 | 1.2 | 1.2 | 1.2 | 1.2 |
| B | Perlite | 0.1 | 0.1 | 0.1 | 0.1 | - | 0.1 | 0.1 | 1.1 |
| | Silica silylate | 0.5 | 0.6 | 0.5 | 0.5 | - | 0.5 | 0.5 | 0.9 |
| | Silica (and) titanium dioxide | 0.1 | 0.1 | 0.1 | 0.1 | - | 0.1 | 0.1 | 3.3 |
| | Silica | - | - | - | - | - | - | - | 0.1 |
| C | Glycerin | 5 | 5.9 | 5 | 5 | 5 | 5 | 5 | 3.3 |
| | Butylene glycol | 8 | 9.4 | 8 | 8 | 8 | 8 | 8 | 2.2 |
| | Phenoxyethanol | 0.5 | 0.6 | 0.5 | 0.5 | 0.5 | 0.5 | 0.5 | 0.5 |
| | Sodium hyaluronate (Mw: 35,000) | 5 | 1.2 | 1 | 1 | 1 | 1 | - | - |
| | Water | qs 100 | qs 100 | qs 100 | qs 100 | qs 100 | qs 100 | qs 100 | qs 100 |
| | Niacinamide | 5 | 5.9 | 5 | 5 | 5 | 5 | 5 | - |
| | Magnesium sulfate | 0.3 | 0.4 | 0.3 | 0.3 | 0.3 | 0.3 | 0.3 | 0.8 |
| D | Ethanol | 6.8 | 8.2 | 6.8 | 6.8 | 6.8 | 6.8 | 6.8 | 7.8 |
| E | Titanium dioxide (and) alumina (and) dimethicone (and) isopropyl titanium triisostearate | 12.6 | - | 12.6 | 12.6 | 12.6 | 12.6 | 12.6 | 12.7 |
| | Iron oxides (and) isopropyl titanium triisostearate | 1.94 | - | 2 | 2 | 2 | 2 | 2 | 2 |
| Water Phase Content (wt%) | | 43 | 50 | 43 | 43 | 43 | 37 | 43 | 37 |
| Stability | | Good | Very good | Fair | Very good | Fair | Very good | Fair | Fair |
| Water-splashing sensation | | Very good | Very good | Very good | Very good | Very good | Fair | Fair | Fair |
| Moist sensation | | Very good | Very good | Very good | Very good | Very good | Very good | Very good | Fair |
| Moisture lastingness | | Very good | Very good | Very good | Bad | Bad | Very good | Very good | Fair |

As shown in Tables 1 and 2 above, the compositions according to Examples 1 to 9 which include the combinations of ingredients (a) to (e) and an aqueous phase in an amount of 40% by weight or more relative to the total weight of the composition showed good stability and sensory properties with respect to "water-splashing sensation", "moist sensation", and "moisture lastingness".

On the other hand, the composition according to Comparative Example 1, which did not include the thickening aid of the present invention, showed inadequate stability. The composition according to Comparative Example 2, which did not include the film-forming polymer, showed poor moisture lastingness. The composition according to Comparative Example 3, which did not include the spherical hydrophobic silica aerogel of the present invention, showed inadequate stability and poor moisture lastingness. The composition according to Comparative Example 4, which includes an aqueous phase less than 40% by weight, did not provide good water-splashing sensation. The composition according to Comparative Example 5, which did not include the anionic polymer, showed inadequate stability and water-splashing sensation. The composition according to Comparative Example 6, which did not include the thickening aid of the present invention and the anionic polymer and includes an aqueous phase less than 40% by weight, showed inadequate properties on all of the aspects.

Therefore, it can be concluded that the composition according to the present invention has a great benefit since it can provide excellent cosmetic properties, such as providing a fresh and moist feeling as well as good moisture lastingness while it remains stable. Therefore the compositions according to the present invention are very useful as cosmetic compositions for keratinous substances, such as skin, preferably skin makeup or skin care compositions, and in particular as foundations.

## Claims

1. A cosmetic emulsion composition having at least one aqueous phase and at least one oily phase, comprising:
(a) at least one spherical hydrophobic silica aerogel;
(b) at least one thickening aid selected from polyoxyalkylenated silicone and alkyl or alkylene carbonate;
(c) at least one film-forming polymer;
(d) at least one polyol in an amount of 6% by weight or more relative to the total weight of the composition; and
(e) at least one anionic polymer,
wherein the aqueous phase is present in an amount of 40% by weight or more relative to the total weight of the composition.

2. The cosmetic composition according to Claim 1, wherein the spherical hydrophobic silica aerogel is a spherical hydrophobic aerogel of silica silylate.

3. The cosmetic composition according to Claim 1 or 2, wherein the spherical hydrophobic silica aerogel has an average circularity determined by an image analysis method of 0.8 or more, and preferably 0.82 or more, and of less than 1, preferably 0.99 or less, more preferably 0.98 or less, even more preferably 0.97 or less, still even more preferably 0.96 or less, and most preferably 0.95 or less.

4. The cosmetic composition according to any one of Claims 1 to 3, wherein the film-forming polymer is selected from silicone resin.

5. The cosmetic composition according to Claim 1 to 4, wherein the film-forming polymer is selected from silicone resin of MQ type.

6. The cosmetic composition according to any one of Claims 1 to 5, wherein the polyol comprises at least one diol and at least one polyol having three or more -OH functions, in particular triol, in combination.

7. The cosmetic composition according to any one of Claims 1 to 6, wherein the polyol comprises at least 3% by weight of at least one diol and at least 3% by weight of at least one polyol having three or more -OH functions, in particular triol, in combination, relative to the total weight of the composition.

8. The cosmetic composition according to any one of Claims 1 to 7, wherein a number average molecular weight of the anionic polymer ranges from 1,000 to 1,000,000, preferably from 2,000 to 500,000, more preferably from 3,000 to 50,000.

9. The cosmetic composition according to any one of Claims 1 to 8, wherein the anionic polymer is selected from the group consisting of polysaccharides such as alginic acid, hyaluronic acid and derivatives thereof, and cellulose polymers (e.g., carboxymethylcellulose), anionic (co)polyaminoacids such as (co)polyglutamic acids, (co)poly(meth)acrylic acids, (co)polyamic acids, (co)polystyrene sulfonate, (co)poly(vinyl sulfate), dextran sulfate, chondroitin sulfate, (co)polymaleic acids, (co)polyfumaric acids, maleic acid (co)polymers, and salts thereof.

10. The cosmetic composition according to any one of Claims 1 to 9, wherein the anionic polymer is selected from hyaluronic acid and derivatives thereof, and a salt thereof; and preferably hyaluronic acid and acetylated hyaluronic acid, and a salt thereof.

11. The cosmetic composition according to any one of Claims 1 to 10, wherein the thickening aid is selected from dimethicone copolyols comprising polyethyleneoxy moiety.

12. The cosmetic composition according to any one of Claims 1 to 11, wherein the thickening aid is selected from alkyl or alkylene carbonate wherein the alkylene chain(s) of the alkylene carbonate(s) and/or the alkyl radical(s) of the alkyl carbonate(s) comprise(s) from 1 to 6 carbon atoms.

13. The cosmetic composition according to any one of Claims 1 to 12, wherein the aqueous phase comprises at least one monoalcohol.

14. The cosmetic composition according to any one of Claims 1 to 13, wherein the cosmetic composition is a skin makeup or a skin care composition, preferably a skin makeup composition, and more preferably a foundation.

15. A cosmetic process for a keratin substance such as skin, comprising applying the cosmetic composition according to any one of Claims 1 to 14 to the keratin substance.

## Patentansprüche

1. Eine kosmetische Emulsionszusammensetzung mit mindestens einer wässrigen Phase und mindestens einer Ölphase, umfassend:
(a) mindestens ein sphärisches hydrophobes Siliciumdioxidaerogel;
(b) mindestens ein Verdickungsmittel, das ausgewählt ist aus polyoxyalkyleniertem Silicon und Alkyl- oder Alkylencarbonat;
(c) mindestens ein filmbildendes Polymer;
(d) mindestens ein Polyol in einer Menge von 6 Gew.-% oder mehr bezogen auf das Gesamtgewicht der Zusammensetzung; und
(e) mindestens ein anionisches Polymer,
wobei die wässrige Phase in einer Menge von 40 Gew.-% oder mehr vorliegt, bezogen auf das Gesamtgewicht der Zusammensetzung.

2. Die kosmetische Zusammensetzung nach Anspruch 1, wobei das sphärische hydrophobe Siliciumdioxidaerogel ein sphärisches hydrophobes Aerogel von Siliciumdioxidsilylat ist.

3. Die kosmetische Zusammensetzung nach Anspruch 1 oder 2, wobei das sphärische hydrophobe Siliciumdioxidaerogel eine durchschnittliche, nach einem Bildanalyseverfahren ermittelte Zirkularität von 0,8 oder mehr, und vorzugsweise von 0,82 oder mehr, und von weniger als 1, vorzugsweise von 0,99 oder weniger, mehr bevorzugt von 0,98 oder weniger, sogar mehr bevorzugt von 0,97 oder weniger, sogar noch mehr bevorzugt von 0,96 oder weniger, und am meisten bevorzugt von 0,95 oder weniger besitzt.

4. Die kosmetische Zusammensetzung nach einem der Ansprüche 1 bis 3, wobei das filmbildende Polymer aus Siliconharz ausgewählt ist.

5. Die kosmetische Zusammensetzung nach Anspruch 1 bis 4, wobei das filmbildende Polymer aus Siliconharz vom MQ-Typ ausgewählt ist.

6. Die kosmetische Zusammensetzung nach einem der Ansprüche 1 bis 5, wobei das Polyol mindestens ein Diol und mindestens ein Polyol mit drei oder mehr -OH-Funktionen, insbesondere Triol, in Kombination umfasst.

7. Die kosmetische Zusammensetzung nach einem der Ansprüche 1 bis 6, wobei das Polyol mindestens 3 Gew.-% von mindestens einem Diol und mindestens 3 Gew.-% von mindestens einem Polyol mit drei oder mehr -OH-Funktionen, insbesondere Triol, in Kombination umfasst, bezogen auf das Gesamtgewicht der Zusammensetzung.

8. Die kosmetische Zusammensetzung nach einem der Ansprüche 1 bis 7, wobei ein zahlengemitteltes Molekulargewicht des anionischen Polymers im Bereich von 1.000 bis 1.000.000 liegt, vorzugsweise im Bereich von 2.000 bis 500.000, mehr bevorzugt im Bereich von 3.000 bis 50.000.

9. Die kosmetische Zusammensetzung nach einem der Ansprüche 1 bis 8, wobei das anionische Polymer ausgewählt ist aus der Gruppe bestehend aus Polysacchariden wie zum Beispiel Alginsäure, Hyaluronsäure und Derivate davon, und aus Cellulosepolymeren (z.B. Carboxymethylcellulose), anionischen (Co-)Polyaminosäuren wie zum Beispiel (Co-)Polyglutaminsäuren, (Co-)Poly(meth)acrylsäuren, (Co-)Polyaminsäuren, (Co-)Polystyrolsulfonat, (Co-)Poly(vinylsulfat), Dextransulfat, Chondroitinsulfat, (Co-)Polymaleinsäuren, (Co-)Polyfumarsäuren, Maleinsäure(co-)polymeren, und Salzen davon.

10. Die kosmetische Zusammensetzung nach einem der Ansprüche 1 bis 9, wobei das anionische Polymer ausgewählt ist aus Hyaluronsäure und Derivaten davon, und einem Salz davon; und vorzugsweise aus Hyaluronsäure und acetylierter Hyaluronsäure, und einem Salz davon.

11. Die kosmetische Zusammensetzung nach einem der Ansprüche 1 bis 10, wobei das Verdickungsmittel ausgewählt ist aus Dimethiconcopolyolen mit einem Polyethylenoxyrest.

12. Die kosmetische Zusammensetzung nach einem der Ansprüche 1 bis 11, wobei das Verdickungsmittel ausgewählt ist aus Alkyl- oder Alkylencarbonat, wobei die Alkylenkette(n) des Alkylencarbonats bzw. der Alkylencarbonate und/oder der Alkylrest bzw. die Alkylreste des Alkylcarbonats bzw. der Alkylcarbonate von 1 bis 6 Kohlenstoffatome umfasst bzw. umfassen.

13. Die kosmetische Zusammensetzung nach einem der Ansprüche 1 bis 12, wobei die wässrige Phase mindestens einen Monoalkohol umfasst.

14. Die kosmetische Zusammensetzung nach einem der Ansprüche 1 bis 13, wobei die kosmetische Zusammensetzung eine Hautschmink- oder eine Hautpflegezusammensetzung ist, vorzugsweise eine Hautschminkzusammensetzung, und mehr bevorzugt eine Grundierung.

15. Ein kosmetisches Verfahren für eine Keratinsubstanz wie zum Beispiel Haut, bei dem die kosmetische Zusammensetzung nach einem der Ansprüche 1 bis 14 auf die Keratinsubstanz aufgebracht wird.

## Revendications

1. Composition cosmétique en émulsion ayant au moins une phase aqueuse et au moins une phase huileuse, comprenant :
(a) au moins un aérogel de silice hydrophobe sphérique ;
(b) au moins une aide à l'épaississement choisie parmi du silicone polyoxyalkyléné et du carbonate d'alkyle ou d'alkylène ;
(c) au moins un polymère filmogène ;
(d) au moins un polyol selon une quantité de 6 % en poids ou plus par rapport au poids total de la composition ; et
(e) au moins un polymère anionique,
dans lequel la phase aqueuse est présente selon une quantité de 40 % en poids ou plus par rapport au poids total de la composition.

2. Composition cosmétique selon la revendication 1, dans laquelle l'aérogel de silice hydrophobe sphérique est un aérogel hydrophobe sphérique de silylate de silice.

3. Composition cosmétique selon la revendication 1 ou 2, dans laquelle l'aérogel de silice hydrophobe sphérique présente une circularité moyenne déterminée par un procédé d'analyse d'image de 0,8 ou plus, et de préférence de 0,82 ou plus, et de moins de 1, de préférence de 0,99 ou moins, de manière plus préférée de 0,98 ou moins, de manière encore plus préférée de 0,97 ou moins, de manière encore davantage plus préférée de 0,96 ou moins, et de manière la plus préférée de 0,95 ou moins.

4. Composition cosmétique selon l'une quelconque des revendications 1 à 3, dans laquelle le polymère filmogène est choisi parmi une résine de silicone.

5. Composition cosmétique selon les revendications 1 à 4, dans laquelle le polymère filmogène est choisi parmi une résine de silicone de type MQ.

6. Composition cosmétique selon l'une quelconque des revendications 1 à 5, dans laquelle le polyol comprend au moins un diol et au moins un polyol ayant trois fonctions -OH ou plus, en particulier triol, en combinaison.

7. Composition cosmétique selon l'une quelconque des revendications 1 à 6, dans laquelle le polyol comprend au moins 3 % en poids d'au moins un diol et au moins 3 % en poids d'au moins un polyol ayant trois fonctions -OH ou plus, en particulier triol, en combinaison, par rapport au poids total de la composition.

8. Composition cosmétique selon l'une quelconque des revendications 1 à 7, dans laquelle un poids moléculaire moyen en nombre du polymère anionique se trouve dans la plage de 1 000 à 1 000 000, de préférence de 2 000 à 500 000, de manière plus préférée de 3 000 à 50 000.

9. Composition cosmétique selon l'une quelconque des revendications 1 à 8, dans laquelle le polymère anionique est choisi parmi le groupe constitué de polysaccharides tels que de l'acide alginique, acide hyaluronique et dérivés de ceux-ci, et polymères de cellulose (par exemple, carboxyméthylcellulose), (co)polyaminoacides anioniques tels que des acides (co)polyglutamiques, acides (co)poly(méth)acryliques, acides (co)polyamiques, sulfonate de (co)polystyrène, (co)poly(sulfate de vinyle), sulfate de dextrane, sulfate de chondroïtine, acides (co)polymaléiques, acides (co)polyfumariques, (co)polymères d'acide maléique, et des sels de ceux-ci.

10. Composition cosmétique selon l'une quelconque des revendications 1 à 9, dans laquelle le polymère anionique est choisi parmi l'acide hyaluronique et des dérivés de celui-ci, et un sel de celui-ci ; et de préférence parmi l'acide hyaluronique et l'acide hyaluronique acétylé, et un sel de ceux-ci.

11. Composition cosmétique selon l'une quelconque des revendications 1 à 10, dans laquelle l'aide à l'épaississement est choisie parmi des copolyols de diméthicone comprenant une fraction polyéthylèneoxy.

12. Composition cosmétique selon l'une quelconque des revendications 1 à 11, dans laquelle l'aide à l'épaississement est choisie parmi du carbonate d'alkyle ou d'alkylène, dans laquelle la ou les chaînes alkylène du ou des carbonates d'alkylène et/ou le ou les radicaux alkyle du ou des carbonates d'alkyle comprennent de 1 à 6 atomes de carbone.

13. Composition cosmétique selon l'une quelconque des revendications 1 à 12, dans laquelle la phase aqueuse comprend au moins un mono-alcool.

14. Composition cosmétique selon l'une quelconque des revendications 1 à 13, dans laquelle la composition cosmétique est un maquillage pour la peau ou une composition de soin de la peau, de préférence une composition de maquillage pour la peau, et de manière plus préférée un fond de teint.

15. Procédé cosmétique pour une substance kératineuse telle que la peau, comprenant l'application de la composition cosmétique selon l'une quelconque des revendications 1 à 14 sur la substance kératineuse.
